# EUROPEAN PATENT APPLICATION

(11) **EP 4 273 159 A1**
(43) Date of publication of application: **08.11.2023**
(21) Application number: 21914252.8
(22) Date of filing: 27.12.2021
(51) Int. Cl.: C07K 14/605, C07K 19/00, C12N 15/11, C12N 15/63, A61K 38/26, A61P 3/04, A61P 3/10

(54) **HUMAN GLP-1 POLYPEPTIDE VARIANT AND USE THEREOF**

(30) Priority: 29.12.2020 CN 202011605173
(71) Applicant: Suzhou Alphamab Co., Ltd, Jiangsu 215000 (CN)
(72) Inventor: XU, Ting, Suzhou, Jiangsu 215000 (CN); JIN, Yuhao, Suzhou, Jiangsu 215000 (CN); WANG, Xiaoxiao, Suzhou, Jiangsu 215000 (CN); WANG, Pilin, Suzhou, Jiangsu 215000 (CN); LI, Qian, Suzhou, Jiangsu 215000 (CN)
(74) Representative: Henderson, Helen Lee
(86) International application number: PCT/CN2021/141608
(87) International publication number: WO 2022/143516

(57) **Abstract**

Provided in the present application is a human GLP-1 polypeptide variant, and a fusion protein or an immunoconjugate constructed by means of using the human GLP-1 polypeptide variant can be used for preventing and/or treating metabolic diseases or conditions related to GLP-1.

## Description

### TECHNICAL FIELD

The present application relates to the field of biomedicine and particularly to a human GLP-1 polypeptide variant and use thereof.

### BACKGROUND

Glucagon-like peptide-1 (GLP-1) is an incretin secreted by L-cells of the small intestinal epithelium. In healthy individuals, GLP-1 is capable of promoting insulin release and inhibiting glucagon secretion in response to the uptake of nutrients by the body. In patients with type II diabetes, administration of a supraphysiological dose of GLP-1 can restore endogenous insulin secretion and lower blood sugar.

The plasma half-life of native GLP-1 is short due to the enzymolysis by dipeptidyl peptidase IV (DPPIV) and renal clearance. Although several GLP-1R agonists modified to prolong the half-life (e.g., dulaglutide) have been developed for the treatment of type II diabetes, there is still a need to develop an effective long-term treatment regimen while maintaining minimal side effects.

### SUMMARY

The present application provides a human GLP-1 polypeptide variant, wherein the amino acid sequence of the human GLP-1 polypeptide variant comprises at least 2 amino acid mutations compared to the amino acid sequence set forth in SEQ ID NO: 2, the at least 2 amino acid mutations being at amino acid positions selected from the group consisting of W31, K26, Q23 and Y19. The present application also provides a fusion protein or immunoconjugate comprising the human GLP-1 polypeptide variant, the fusion protein or immunoconjugate having at least one of the following properties: (1) ability to bind to the GLP-1 receptor, (2) ability to activate the GLP-1 receptor and thereby improve the cAMP level, (3) relatively long blood half-life, (4) relatively high stability *in vivo* and *in vitro,* (5) relatively high plasma concentration, and (6) ability to lower blood sugar.

In one aspect, the present application provides a human GLP-1 polypeptide variant, wherein the amino acid sequence of the human GLP-1 polypeptide variant comprises at least 2 amino acid mutations compared to the amino acid sequence set forth in SEQ ID NO: 2, the at least 2 amino acid mutations being at amino acid positions selected from the group consisting of W31, K26, Q23 and Y19.

In certain embodiments, the human GLP-1 polypeptide variant has at least partial activity of human GLP-1.

In certain embodiments, the amino acid sequence of the human GLP-1 polypeptide variant comprises at least 2 mutations at amino acid positions selected from the group consisting of W31, K26 and Y19 compared to the amino acid sequence set forth in SEQ ID NO: 2.

In certain embodiments, the amino acid sequence of the human GLP-1 polypeptide variant comprises at least 2 mutations at amino acid positions selected from the group consisting of W31, K26 and Q23 compared to the amino acid sequence set forth in SEQ ID NO: 2.

In certain embodiments, the amino acid sequence of the human GLP-1 polypeptide variant comprises mutations at amino acid positions W31 and K26 compared to the amino acid sequence set forth in SEQ ID NO: 2.

In certain embodiments, the amino acid sequence of the human GLP-1 polypeptide variant comprises mutations at amino acid positions W31 and Q23 compared to the amino acid sequence set forth in SEQ ID NO: 2.

In certain embodiments, the amino acid sequence of the human GLP-1 polypeptide variant comprises mutations at amino acid positions W31 and Y19 compared to the amino acid sequence set forth in SEQ ID NO: 2.

In certain embodiments, the amino acid sequence of the human GLP-1 polypeptide variant comprises mutations at amino acid positions K26 and Q23 compared to the amino acid sequence set forth in SEQ ID NO: 2.

In certain embodiments, the amino acid sequence of the human GLP-1 polypeptide variant comprises mutations at amino acid positions K26 and Y19 compared to the amino acid sequence set forth in SEQ ID NO: 2.

In certain embodiments, the amino acid sequence of the human GLP-1 polypeptide variant comprises mutations at amino acid positions W31, K26 and Y19 compared to the amino acid sequence set forth in SEQ ID NO: 2.

In certain embodiments, the amino acid sequence of the human GLP-1 polypeptide variant comprises mutations at amino acid positions W31, K26 and Q23 compared to the amino acid sequence set forth in SEQ ID NO: 2.

In certain embodiments, the human GLP-1 polypeptide variant comprises an amino acid substitution at the position W31, and the amino acid substitution is W31Y, W31K, W31R or W31A. For example, the amino acid substitution is W31Y, W31K or W31R. As another example, the amino acid substitution is W31Y.

In certain embodiments, the human GLP-1 polypeptide variant comprises an amino acid substitution at the position Y19, and the amino acid substitution is selected from any one of the following amino acid substitutions: Y19A, Y19L, Y19T, Y19F, Y19I, Y19V and Y19S.

In certain embodiments, the human GLP-1 polypeptide variant comprises an amino acid substitution at the position Q23, and the amino acid substitution is selected from any one of the following amino acid substitutions: Q23E, Q23T, Q23S and Q23N.

In certain embodiments, the human GLP-1 polypeptide variant comprises an amino acid substitution at the position K26, and the amino acid substitution is K26R.

In certain embodiments, the human GLP-1 polypeptide variant comprises an amino acid sequence set forth in any one of SEQ ID NOs: 29, 31-53 and 61-71. In certain embodiments, the human GLP-1 polypeptide variant comprises an amino acid sequence set forth in any one of SEQ ID NOs: 29, 32-53 and 61-71. In certain embodiments, the fusion protein or immunoconjugate comprises an amino acid sequence set forth in SEQ ID NO: 29. In certain embodiments, the fusion protein or immunoconjugate comprises an amino acid sequence set forth in any one of SEQ ID NOs: 32-53. In certain embodiments, the fusion protein or immunoconjugate comprises an amino acid sequence set forth in any one of SEQ ID NOs: 61-71.

In another aspect, the present application provides a fusion protein or immunoconjugate comprising the human GLP-1 polypeptide variant.

In certain embodiments, the fusion protein or immunoconjugate further comprises an immunoglobulin Fc region.

In certain embodiments, the immunoglobulin Fc region of the fusion protein or immunoconjugate is an Fc region derived from IgG.

In certain embodiments, the immunoglobulin Fc region of the fusion protein or immunoconjugate is an Fc region derived from IgG4.

In certain embodiments, the immunoglobulin Fc region comprises an amino acid sequence set forth in any one of SEQ ID NOs: 139-143.

In certain embodiments, the human GLP-1 polypeptide variant is fused in-frame with the immunoglobulin Fc region.

In certain embodiments, the fusion protein or immunoconjugate comprises a linker peptide.

In certain embodiments, the human GLP-1 polypeptide variant and the immunoglobulin Fc region are linked through the linker peptide.

In certain embodiments, the linker peptide comprises an amino acid sequence set forth in any one of SEQ ID NOs: 144-149.

In certain embodiments, the human GLP-1 polypeptide variant, the linker peptide and the immunoglobulin Fc region are fused in-frame.

In certain embodiments, the fusion protein or immunoconjugate comprises an amino acid sequence set forth in any one of SEQ ID NOs: 96, 98-120 and 128-138. In certain embodiments, the fusion protein or immunoconjugate comprises an amino acid sequence set forth in any one of SEQ ID NOs: 96, 97-120 and 128-138. In certain embodiments, the fusion protein or immunoconjugate comprises an amino acid sequence set forth in SEQ ID NO: 96. In certain embodiments, the fusion protein or immunoconjugate comprises an amino acid sequence set forth in any one of SEQ ID NOs: 99-122. In certain embodiments, the fusion protein or immunoconjugate comprises an amino acid sequence set forth in any one of SEQ ID NOs: 128-138.

In another aspect, the present application provides an isolated nucleic acid molecule encoding the human GLP-1 polypeptide variant or the fusion protein or immunoconjugate.

In another aspect, the present application provides a vector comprising the isolated nucleic acid molecule.

In another aspect, the present application provides a cell comprising or expressing the human GLP-1 polypeptide variant, the fusion protein or immunoconjugate, the isolated nucleic acid molecule, or the vector.

In another aspect, the present application provides a pharmaceutical composition comprising the human GLP-1 polypeptide variant, the fusion protein or immunoconjugate, the isolated nucleic acid molecule, the vector, or the cell, and optionally a pharmaceutically acceptable adjuvant.

In another aspect, the present application provides use of the human GLP-1 polypeptide variant or the fusion protein or immunoconjugate for preparing a medicament for preventing and/or treating metabolic diseases or conditions.

In another aspect, the present application provides a method for preventing and/or treating metabolic diseases or conditions, the method comprising administering the human GLP-1 polypeptide variant or the fusion protein or immunoconjugate.

In another aspect, the present application provides the human GLP-1 polypeptide variant or the fusion protein or immunoconjugate for use in the prevention and/or treatment of metabolic diseases or conditions.

In certain embodiments, the metabolic diseases or conditions include GLP-1-associated metabolic conditions.

In certain embodiments, the metabolic diseases or conditions include diabetes.

In another aspect, the present application provides a method for increasing or promoting insulin expression in a subject in need thereof, the method comprising administering to the subject an effective amount of the human GLP-1 polypeptide variant or the fusion protein or immunoconjugate. In another aspect, the present application provides the human GLP-1 polypeptide variant or the fusion protein or immunoconjugate for use in increasing or promoting insulin expression in a subject in need thereof.

In another aspect, the present application provides use of the human GLP-1 polypeptide variant or the fusion protein or immunoconjugate in the preparation of a medicament for increasing or promoting insulin expression in a subject in need thereof.

Other aspects and advantages of the present application will be readily apparent to those skilled in the art from the following detailed description. Only exemplary embodiments of the present application are shown and described in the following detailed description. As those skilled in the art will recognize, the content of the present application enables those skilled in the art to make changes to the specific embodiments disclosed without departing from the spirit and scope of the invention involved in the present application. Accordingly, descriptions in the drawings and specification are only illustrative rather than restrictive.

### BRIEF DESCRIPTION OF THE DRAWINGS

Specific features of the invention involved in the present application are set forth in appended claims. Features and advantages of the invention involved in the present application will be better understood by reference to the exemplary embodiments and drawings described in detail below. A brief description of the drawings is as below.
FIG. 1 shows the binding activity of fusion proteins GM-WY and GM-FY to the GLP-1 receptor.
FIG. 2 shows the binding activity of fusion protein GM-WY to the GLP-1 receptor.
FIG. 3 shows the binding activity of fusion proteins GM-WR and GM-FH to the GLP-1 receptor.
FIG. 4 shows the binding activity of fusion proteins GM-WY, GM-QN, GM-QT, GM-QS, GM-YL, GM-YT and GM-YF to the GLP-1 receptor.
FIG. 5 shows the binding activity of fusion proteins GM-WY, GM-YI, GM-YV, GM-YS, GM-QE and GM-YA to the GLP-1 receptor.
FIG. 6 shows the binding activity of fusion proteins GM-FT, GM-FN, GM-FS and GM-FQ to the GLP-1 receptor.
FIG. 7 shows the binding activity of fusion proteins GM-FE, GM-FV, GM-FK and GM-FI to the GLP-1 receptor.
FIG. 8 shows the binding activity of fusion proteins GM-WY, GM-KRFY, GM-KRWY and GM-KRWYFY to the GLP-1 receptor.
FIG. 9 shows the binding activity of fusion proteins GM-WY, GM-KRQN, GM-KRQT, GM-KRQS, GM-KRYL, GM-KRYT and GM-KRYF to the GLP-1 receptor.
FIG. 10 shows the binding activity of fusion proteins GM-KRWY, GM-KRYI, GM-KRYV, GM-KRYS, GM-KRQE and GM-KRYA to the GLP-1 receptor.
FIG. 11 shows the binding activity of fusion proteins GM-WY, GM-WYQN, GM-WYQS and GM-WYQT to the GLP-1 receptor.
FIG. 12 shows the binding activity of fusion proteins GM-WY, GM-WYYL, GM-WYYT, GM-WYYF, GM-WYYI, GM-WYYV and GM-WYYS to the GLP-1 receptor.
FIG. 13 shows the binding activity of fusion proteins GM-WYYA and GM-WYQE to the GLP-1 receptor.
FIGs. 14A-14B show the activation of the cAMP/PKA signaling pathway by fusion protein GM-WYYA at 6 h (A) and at 24 h (B).
FIGs. 15A-15B show the activation of the cAMP/PKA signaling pathway by fusion protein GM-WYQE at 6 h (A) and at 24 h (B).
FIG. 16 shows the binding activity of fusion proteins GM-WYFL and GM-WY to the GLP-1 receptor.
FIG. 17 shows the binding activity of fusion proteins GM-WIFL, GM-WA, GM-WL and GM-WLFL to the GLP-1 receptor.
FIG. 18 shows the binding activity of fusion proteins GM-KRWYYF, GM-KRWYYL, GM-KRWYYT, GM-KRWYQN, GM-KRWYQT and GM-KRWYQS to the GLP-1 receptor.
FIG. 19 shows the binding activity of fusion proteins GM-KRWYYI, GM-KRWYYV, GM-KRWYYS, GM-KRWYQE and GM-KRWYYA to the GLP-1 receptor.
FIG. 20 shows the binding activity of fusion proteins GM-KRWYYA and GM-KRWYQN to the GLP-1 receptor.
FIGs. 21A-21B show the activation of the cAMP/PKA signaling pathway by fusion proteins GM-KRWY and GM-KRWYQE at 6 h (A) and at 24 h (B).
FIGs. 22A-22B show the activation of the cAMP/PKA signaling pathway by fusion proteins GM-KRWYYA and GM-KRWYYL at 6 h (A) and at 24 h (B).
FIGs. 23A-23B show the activation of the cAMP/PKA signaling pathway by fusion proteins GM-KRWYYS and GM-KRWYYI at 6 h (A) and at 24 h (B).
FIGs. 24A-24B show the activation of the cAMP/PKA signaling pathway by fusion proteins GM-KRWYYT and GM-KRWYQN at 6 h (A) and at 24 h (B).
FIGs. 25A-25B show the activation of the cAMP/PKA signaling pathway by fusion proteins GM-KRWYQT and GM-KRWYQS at 6 h (A) and at 24 h (B).
FIGs. 26A-26B show the activation of the cAMP/PKA signaling pathway by fusion proteins GM-KRWYYA and GM-KRWYQN at 6 h (A) and at 24 h (B).
FIG. 27 shows the pharmacokinetic effect of fusion protein GM-KRWY.
FIG. 28 shows the pharmacokinetic effect of fusion proteins GM-KRWYYL, GM-KRWYYI and GM-KRWYYA.
FIG. 29 shows the pharmacokinetic effect of fusion proteins GM-KRWYQN, GM-KRWYQE and GM-KRWYYA.
FIGs. 30A-30B show the hypoglycemic activity of fusion protein GM-KRWY in an oral glucose tolerance test.
FIGs. 31A-31B show the hypoglycemic activity of fusion proteins GM-KRWYQE and GM-KRWYYA in oral glucose tolerance tests.
FIG. 32 shows an example of the amino acid sequence numbering in the present application.
FIG. 33 shows the binding activity of fusion proteins GM-KRWY, GM-KAWA, GM-KAWY and GM-KRWA to the GLP-1 receptor.

### DETAILED DESCRIPTION

The embodiments of the present invention are described below with reference to specific examples, and other advantages and effects of the present invention will be readily apparent to those skilled in the art from the disclosure of the present specification.

### Definitions of Terms

In the present application, the term "GLP-1" generally refers to glucagon-like peptide-1. The native GLP-1 molecule undergoes processing *in vivo,* during which the first 6 amino acids are removed by cleaving. Therefore, it is customary in the art to refer to the first amino acid at the N-terminus of the amino acid sequence of GLP-1 as being at position 7 and the last amino acid at the C-terminus as being at position 37. The processed peptide can be further modified *in vivo* by removing the C-terminal glycine residue and replacing it with an amide group. GLP-1 is usually found in two biologically active forms: GLP-1(7-37)OH and GLP-1 (7-36)NH₂. The "GLP-1" described herein includes native, artificially synthesized or modified GLP-1 proteins and also intact GLP-1 protein or functional fragments thereof, as well as different biologically active forms of GLP-1 protein. For example, wild-type human GLP-1 may comprise an amino acid sequence set forth in SEQ ID NO: 1, where the N-terminal amino acid residue H is referred to as being at position 7. Accordingly, the term "K26" in the present application generally refers to the position of the amino acid at position 26, counting from the N-terminal H at position 7 of GLP-1 protein; in the amino acid sequence set forth in SEQ ID NO: 1, the amino acid at position 26 is K; the term "W31" generally refers to the position of the amino acid at position 31, counting from the N-terminal H at position 7 of GLP-1 protein; in the amino acid sequence set forth in SEQ ID NO: 1, the amino acid at position 31 is W.

The human GLP-1 may be engineered, and the GLP-1 variant produced by engineering may have at least partial activity of the original human GLP-1. For example, an exemplary engineered wild-type human GLP-1 may have an amino acid sequence set forth in SEQ ID NO: 2.

In the present application, the term "at least partial activity of human GLP-1" generally refers to having one or more activities of human GLP-1 protein, or at least 20% (e.g., at least 25%, 30%, 35%, 40%, 45%, or 50% or more) of the activity of human GLP-1 protein. In the term "at least partial activity of human GLP-1", the human GLP-1 may be the wild type, e.g., the amino acid sequence set forth in SEQ ID NO: 1. In the term "at least partial activity of human GLP-1", the human GLP-1 may be a GLP-1 variant produced by engineering wild-type human GLP-1, e.g., the amino acid sequence set forth in SEQ ID NO: 2. For example, the human GLP-1 polypeptide variant of the present application may have at least partial activity of human GLP-1 which has an amino acid sequence set forth in SEQ ID NO: 1. In some other cases, the human GLP-1 polypeptide variant of the present application may have at least partial activity of human GLP-1 which has an amino acid sequence set forth in SEQ ID NO: 2.

The activity need not be at the same level as the activity of the human GLP-1 protein. It may be similar to, or higher or lower than the activity of the human GLP-1 protein. In certain cases, "at least partial activity of human GLP-1" may refer to one or more selected from the group consisting of: the activity of binding to the GLP-1 receptor, the activity of activating the GLP-1 receptor, the activity of activating adenylate cyclase, the activity of promoting an increase in the intracellular cyclic adenosine monophosphate (cAMP) level, the activity of positively modulating the intracellular Ca²⁺ level, the activity of stimulating insulin secretion, the activity of increasing liver glycogen storage, the activity of delaying gastric emptying, the activity of inhibiting gastric motility, the activity of reducing appetite, the activity of inhibiting β-cell apoptosis, the activity of inhibiting postprandial glucagon secretion, the activity of alleviating hypoglycemia, and the activity of reducing body weight. For example, it can be measured by measuring the ability to bind to the GLP-1 receptor or the level of cAMP expression. The "at least partial activity of human GLP-1" may be at least partial activity of a fusion protein which comprises human GLP-1 (e.g., a fusion protein with an Fc region).

In the present application, the term "GLP-1R" generally refers to glucagon-like peptide-1 receptor. GLP-1R binding to GLP-1 can activate a signaling cascade, leading to activation of adenylate cyclase and an increase in intracellular cAMP level. The GLP-1R described herein may include the full-length molecule, variants, fragments, and composites of native GLP-1R, so long as the activity of GLP-1R is retained. For example, the GLP-1R described herein may comprise an amino acid sequence set forth under NCBI database accession No. NP_002053.3.

In the present application, the term "polypeptide" generally refers to molecules composed of monomers (amino acids) linearly linked by amide bonds (also known as peptide bonds). The term "polypeptide" may refer to any chain of two or more amino acids and does not refer to products of particular length. The polypeptide described herein includes peptides, dipeptides, tripeptides, oligopeptides, proteins, amino acid chains, or any other chains used to refer to having two or more amino acids, and the term "polypeptide" may be used in place of or is used interchangeably with any of these terms. The term "polypeptide" may also refer to the product of post-expression modification of a polypeptide, including but not limited to glycosylation, acetylation, phosphorylation, acylation, derivatization by known protective/blocking groups, proteolytic cleavage, or modification by non-naturally occurring amino acids. The polypeptide may be derived from natural biological sources or produced through recombinant technology.

In the present application, the term "variant" generally refers to a protein molecule having sequence homology with a native biologically active protein. The polypeptide variant described herein include polypeptides having altered amino acid sequences by addition (including insertion), deletion, modification and/or substitution of one or more amino acid residues, which differ from the parent sequence while retaining at least one therapeutic and/or biological activity of the parent sequence. For example, the variant may have at least 0%, 65%, 70%, 75%, 80%, 85%, 90%, 95%, 96%, 97%, 98% or 99% sequence identity to the parent protein. The variant may or may not naturally occur. The variant that does not naturally occur can be generated using known techniques in the art. The polypeptide variant may comprise conservative or non-conservative amino acid substitutions, deletions or additions.

In the present application, the term "mutation" generally includes any type of alteration or modification of a sequence (a nucleic acid or an amino acid sequence), including deletion, truncation, inactivation, disruption, substitution or translocation of an amino acid or a nucleotide. In the present application, an amino acid mutation may be an amino acid substitution, and the term "substitution" generally refers to the replacement of at least one amino acid residue in a predetermined parent amino acid sequence with a different "replacement" amino acid residue. The one or more replaced amino acid residues may be "naturally-occurring amino acid residues" (i.e., genetically encoded).

In the present application, the term "hinge region" generally refers to the region between the CH₁ and CH₂ functional regions of the heavy chain of an immunoglobulin. The hinge region is generally derived from IgG; for example, it may be derived from IgG1, IgG2, IgG3 or IgG4.

In the present application, the term "fusion protein" generally refers to a longer polypeptide comprising or consisting of a polypeptide whose amino acid sequence can be fused directly or indirectly (via a linker, e.g., a linker peptide) to the amino acid sequence of a heterologous polypeptide (e.g., a polypeptide unrelated to the previous polypeptide or the domain thereof). In certain cases, the fusion protein may comprise the polypeptide variant and an immunoglobulin Fc region directly or indirectly linked (e.g., via a linker peptide) to the polypeptide variant. For example, the linker peptide may be GS, GAP, G4S, (G4S)2, (G4S)3 or (G4S)4.

In the present application, the term "immunoconjugate" generally refers to a conjugate formed by directly or indirectly linking (e.g., covalently via a linker) a polypeptide or protein to an active substance of interest (e.g., a protein, a nucleic acid, a drug, or a label molecule).

In the present application, the term "immunoglobulin" generally refers to a protein consisting essentially of one or more polypeptides encoded by immunoglobulin genes. Recognized human immunoglobulin genes include κ, λ, α (IgA1 and IgA2), γ (IgG1, IgG2, IgG3, and IgG4), δ, ε and µ constant region genes, as well as a multitude of immunoglobulin variable region genes. Of the "light chain" (about 25 KD and 214 amino acids) of a full-length immunoglobulin, the NH2-terminus (about 110 amino acids) is encoded by a variable region gene and the COOH-terminus is encoded by a κ or λ constant region gene. Generally, the immunoglobulin may be a heterotetrameric glycoprotein of about 150,000 daltons consisting of two identical light (L) chains and two identical heavy (H) chains. A native immunoglobulin consists essentially of two Fab molecules and an Fc region linked by an immunoglobulin hinge region.

In the present application, the term "Fc region" generally refers to the C-terminal region of the heavy chain of an immunoglobulin, which can be produced by digesting an intact antibody with papain. The Fc region may be a native sequence Fc region or a variant Fc region. The Fc region of an immunoglobulin generally comprises 2 constant domains (a CH₂ domain and a CH₃ domain), and may optionally comprise a CH₄ domain.

In the present application, the term "fused in-frame" generally refers to the joining of two or more open reading frames (ORFs) in a manner that maintains the correct reading frame of the original ORFs to form a continuous longer ORF. Thus, the resulting recombinant fusion protein is a single protein comprising two or more segments that correspond to polypeptides encoded by the original ORFs.

In the present application, the term "nucleic acid molecule" generally refers to an isolated form of any length of nucleotides, deoxyribonucleotides or ribonucleotides or analogs thereof, isolated from its natural environment, or artificially synthesized.

In the present application, the term "vector" generally refers to a nucleic acid molecule capable of self-replication in a suitable host cell, which transfers an inserted nucleic acid molecule into a host cell and/or between host cells. The vector may include a vector for primarily inserting DNA or RNA into a cell, a vector for primarily replicating DNA or RNA, and a vector for primarily expressing transcription and/or translation of DNA or RNA. The vector may also include vectors having a variety of the above-described functions. The vector may be a polynucleotide capable of being transcribed and translated into a polypeptide when introduced into a suitable host cell. Generally, the vector can produce the desired expression product by culturing an appropriate host cell comprising the vector. In the present application, the term "cell" generally refers to an individual cell, cell line or cell culture that may comprise or has comprised a plasmid or vector comprising the nucleic acid molecule described herein, or that is capable of expressing the antibody or the antigen-binding fragment thereof described herein. The host cell may comprise progeny of a single host cell. Due to natural, accidental or deliberate mutations, progeny cells may not necessarily be identical in morphology or in genome to the original parent cell, but are capable of expressing the antibody or the antigen-binding fragment thereof described herein. The host cell may be obtained by transfecting cells with the vector described herein *in vitro.* The host cell may be a prokaryotic cell (e.g., *E. coli*) or a eukaryotic cell (e.g., a yeast cell, a COS cell, a Chinese hamster ovary (CHO) cell, a HeLa cell, an HEK293 cell, a COS-1 cell, an NS0 cell, or a myeloma cell). The recombinant host cell includes not only particular cells but also progeny of such cells.

In the present application, the term "metabolic diseases or conditions" generally refers to diseases or conditions that disrupt the normal metabolic processes in the body, rendering the body unable to properly utilize and/or store energy. The metabolic diseases or conditions may refer to diseases of carbohydrate metabolism disorders, diseases of lipid metabolism disorders and/or diseases of mitochondrial disorders. The metabolic diseases or conditions may be associated with diet, toxins or infections, or may be genetic diseases. The metabolic diseases or conditions may include diseases or conditions caused by lack of metabolism-associated enzymes or metabolism-associated enzymatic dysfunction. The metabolic diseases or conditions described herein may be selected from diabetes, obesity and other GLP-1-associated metabolic conditions.

In the present application, "dulaglutide" generally refers to a heterologous fusion protein comprising a GLP-1 analog, which is formed by covalently linking the GLP-1 analog (of which the amino acid sequence is set forth in SEQ ID NO: 2) to an Fc region derived from human IgG4 (of which the amino acid sequence is set forth in SEQ ID NO: 143) by a small-molecule peptide (of which the amino acid sequence is set forth in SEQ ID NO: 148). Dulaglutide is sold under the brand name TRULICITY^{®}. Dulaglutide comprises an amino acid sequence set forth in SEQ ID NO: 4 (see PCT patent WO2009009562 and U.S. Pat. US7452966).

In the present application, the term "comprise", "comprising", "contain" or "containing" is generally intended to include the explicitly specified features without excluding other elements.

In the present application, the term "about" generally means varying by 0.5%-10% above or below the stated value, for example, varying by 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5% or 10% above or below the stated value.

### Detailed Description of the Invention

### Polypeptide variant

In one aspect, the present application provides a human GLP-1 polypeptide variant having at least partial activity of human GLP-1. In certain cases, the human GLP-1 polypeptide variant may have one or more activities selected from the group consisting of: the activity of binding to the GLP-1 receptor, the activity of activating the GLP-1 receptor, the activity of activating adenylate cyclase, the activity of promoting an increase in the intracellular cyclic adenosine monophosphate (cAMP) level, the activity of positively modulating the intracellular Ca²⁺ level, the activity of stimulating insulin secretion, the activity of increasing liver glycogen storage, the activity of delaying gastric emptying, the activity of inhibiting gastric motility, the activity of reducing appetite, the activity of inhibiting β-cell apoptosis, the activity of inhibiting postprandial glucagon secretion, the activity of alleviating hypoglycemia, and the activity of reducing body weight. For example, it can be measured by measuring the ability to bind to the GLP-1 receptor or the level of cAMP expression. The activity of the human GLP-1 polypeptide variant may be at least 20% (e.g., at least 25%, 30%, 35%, 40%, 45%, or 50% or more) of the activity of human GLP-1.

Compared to the amino acid sequence set forth in SEQ ID NO: 2: HGEGTFTSDVSSYLEEQAAKEFIAWLVKGGG (SEQ ID NO: 2), the amino acid sequence of the human GLP-1 polypeptide variant may comprise at least 2 amino acid mutations, e.g., 2, 3, 4 or more amino acid mutations. For example, the amino acid sequence of the human GLP-1 polypeptide variant may comprise 2-4 (e.g., 2-3, 3 or 2) amino acid substitutions compared to the amino acid sequence set forth in SEQ ID NO: 2. The at least 2 amino acid mutations may be at amino acid positions selected from the group consisting of K26, W31, Y19 and Q23.

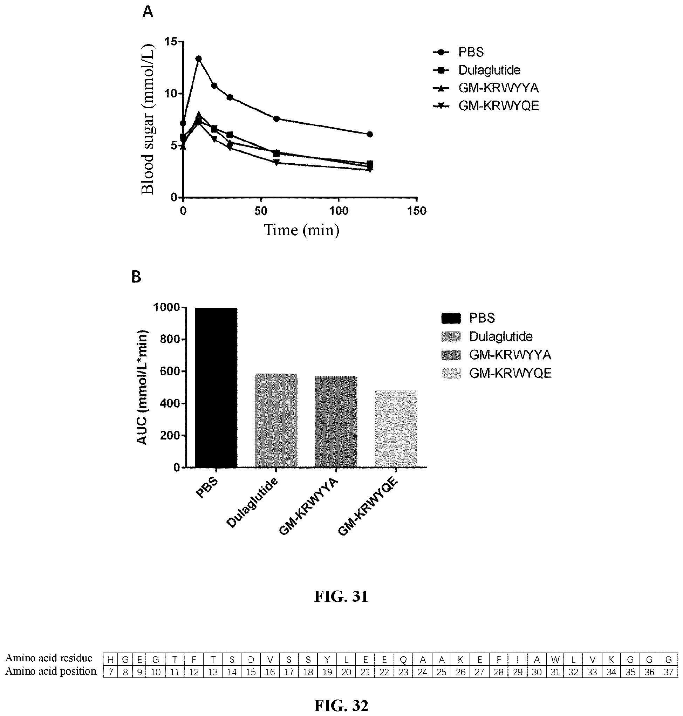

In the present application, the amino acid position "Xn" means that an amino acid substitution occurs at residue X corresponding to position n in the amino acid sequence set forth in SEQ ID NO: 1 or SEQ ID NO: 2, wherein n is a positive integer (for GLP-1, n starts at 7) and X is an abbreviation of any amino acid residue. For example, amino acid position "W31" indicates the position corresponding to the amino acid at position 31 of the amino acid sequence set forth in SEQ ID NO: 1 or SEQ ID NO: 2. For the amino acid sequence set forth in SEQ ID NO: 2, the correspondence of amino acid residue X to position n is shown in FIG. 32.

In the present application, the amino acid sequence of the human GLP-1 polypeptide variant may comprise at least 2 mutations at amino acid positions selected from the group consisting of W31, K26 and Y19 compared to the amino acid sequence set forth in SEQ ID NO: 2.

In the present application, the amino acid sequence of the human GLP-1 polypeptide variant may comprise at least 2 mutations at amino acid positions selected from the group consisting of W31, K26 and Q23 compared to the amino acid sequence set forth in SEQ ID NO: 2.

For example, the amino acid sequence of the human GLP-1 polypeptide variant may comprise 2 amino acid mutations at amino acid positions W31 and K26 compared to the amino acid sequence set forth in SEQ ID NO: 2. For example, the amino acid sequence of the human GLP-1 polypeptide variant has 2 amino acid mutations, which may be at amino acid positions W31 and K26. For example, the human GLP-1 polypeptide variant may comprise an amino acid sequence set forth in SEQ ID NO: 29.

For example, the amino acid sequence of the human GLP-1 polypeptide variant may comprise 2 amino acid mutations at amino acid positions W31 and Y19 compared to the amino acid sequence set forth in SEQ ID NO: 2. For example, the amino acid sequence of the human GLP-1 polypeptide variant has 2 amino acid mutations, which may be at amino acid positions W31 and Y26. For example, the human GLP-1 polypeptide variant may comprise an amino acid sequence set forth in any one of SEQ ID NOs: 47-53.

For example, the amino acid sequence of the human GLP-1 polypeptide variant may comprise 2 amino acid mutations at amino acid positions W31 and Q23 compared to the amino acid sequence set forth in SEQ ID NO: 2. For example, the amino acid sequence of the human GLP-1 polypeptide variant has 2 amino acid mutations, which may be at amino acid positions W31 and Q23. For example, the human GLP-1 polypeptide variant may comprise an amino acid sequence set forth in any one of SEQ ID NOs: 43-46.

For example, the amino acid sequence of the human GLP-1 polypeptide variant may comprise 2 amino acid mutations at amino acid positions K26 and Y19 compared to the amino acid sequence set forth in SEQ ID NO: 2. For example, the amino acid sequence of the human GLP-1 polypeptide variant has 2 amino acid mutations, which may be at amino acid positions K26 and Y19. For example, the human GLP-1 polypeptide variant may comprise an amino acid sequence set forth in any one of SEQ ID NOs: 36-42.

For example, the amino acid sequence of the human GLP-1 polypeptide variant may comprise 2 amino acid mutations at amino acid positions K26 and Q23 compared to the amino acid sequence set forth in SEQ ID NO: 2. For example, the amino acid sequence of the human GLP-1 polypeptide variant has 2 amino acid mutations, which may be at amino acid positions K26 and Q23. For example, the human GLP-1 polypeptide variant may comprise an amino acid sequence set forth in any one of SEQ ID NOs: 32-35.

For example, the amino acid sequence of the human GLP-1 polypeptide variant may comprise 2 amino acid mutations at amino acid positions Y19 and Q23 compared to the amino acid sequence set forth in SEQ ID NO: 2. For example, the amino acid sequence of the human GLP-1 polypeptide variant has 2 amino acid mutations, which may be at amino acid positions Y19 and Q23.

For example, the amino acid sequence of the human GLP-1 polypeptide variant may comprise 3 amino acid mutations at amino acid positions W31, K26 and Y19 compared to the amino acid sequence set forth in SEQ ID NO: 2. For example, the amino acid sequence of the human GLP-1 polypeptide variant has 3 amino acid mutations, which may be at amino acid positions W31, K26 and Y19.

For example, the amino acid sequence of the human GLP-1 polypeptide variant may comprise 3 amino acid mutations at amino acid positions W31, K26 and Q23 compared to the amino acid sequence set forth in SEQ ID NO: 2. For example, the amino acid sequence of the human GLP-1 polypeptide variant has 3 amino acid mutations, which may be at amino acid positions W31, K26 and Q23.

For example, the amino acid sequence of the human GLP-1 polypeptide variant may comprise 3 amino acid mutations at amino acid positions Y19, K26 and Q23 compared to the amino acid sequence set forth in SEQ ID NO: 2. For example, the amino acid sequence of the human GLP-1 polypeptide variant has 3 amino acid mutations, which may be at amino acid positions Y19, K26 and Q23.

For example, the amino acid sequence of the human GLP-1 polypeptide variant may comprise 3 amino acid mutations at amino acid positions W31, Y19 and Q23 compared to the amino acid sequence set forth in SEQ ID NO: 2. For example, the amino acid sequence of the human GLP-1 polypeptide variant has 3 amino acid mutations, which may be at amino acid positions W31, Y19 and Q23.

For example, the amino acid sequence of the human GLP-1 polypeptide variant may comprise 4 amino acid mutations at amino acid positions W31, K26, Y19 and Q23 compared to the amino acid sequence set forth in SEQ ID NO: 2. For example, the amino acid sequence of the human GLP-1 polypeptide variant has 4 amino acid mutations, which may be at amino acid positions W31, K26, Y19 and Q23.

In the present application, the amino acid mutations may include amino acid substitutions. The amino acid mutations cause the GLP-1 polypeptide variant to retain at least partial activity of human GLP-1 (e.g., a human GLP-1 protein comprising an amino acid sequence set forth in any one of SEQ ID NOs: 1 and 2). The amino acid mutations may cause a fusion protein of the GLP-1 polypeptide variant to retain at least partial activity of a fusion protein of human GLP-1 (e.g., a human GLP-1 protein comprising an amino acid sequence set forth in any one of SEQ ID NOs: 1 and 2). The amino acid mutations may cause a fusion protein of the GLP-1 polypeptide variant and an Fc region to retain at least partial activity of a fusion protein of human GLP-1 (e.g., a human GLP-1 protein comprising an amino acid sequence set forth in any one of SEQ ID NOs: 1 and 2) and the Fc region.

In the present application, an amino acid substitution may be comprised at the position K26, and the amino acid substitution may be K26R.

In the present application, an amino acid substitution may be comprised at the position W31, and the amino acid substitution may be W31Y, W31A, W31R or W31K. In the present application, an amino acid substitution may be comprised at the position W31, and the amino acid substitution may be W31Y, W31K or W31R. For example, an amino acid substitution may be comprised at the position W31, and the amino acid substitution may be W31Y.

In the present application, an amino acid substitution may be comprised at the position Y19, and the amino acid substitution may be Y19A, Y19L, Y19T, Y19F, Y19I, Y19V or Y19S.

In the present application, an amino acid substitution may be comprised at the position Q23, and the amino acid substitution may be Q23E, Q23T, Q23S or Q23N.

In the present application, the amino acid substitution "XnY" means that residue X corresponding to position n in the amino acid sequence set forth in SEQ ID NO: 1 or SEQ ID NO: 2 is substituted with amino acid residue Y, wherein n is a positive integer (for GLP-1, n starts at 7); X and Y are each independently an abbreviation of any amino acid residue and X differs from Y. For example, the amino acid substitution "W31Y" means that the amino acid residue W corresponding to position 31 in the amino acid sequence set forth in SEQ ID NO: 1 or SEQ ID NO: 2 is substituted with the amino acid residue Y.

In the present application, the amino acid sequence of the human GLP-1 polypeptide variant may comprise at least 2 amino acid substitutions compared to the amino acid sequence set forth in SEQ ID NO: 1 or SEQ ID NO: 2, and the amino acid substitutions may be selected from the group consisting of: 1) K26R, 2) any one of W31Y, W31R, W31K and W31A, 3) any one of Y19A, Y19L, Y19T, Y19F, Y19I, Y19V and Y19S, and 4) any one of Q23N, Q23T, Q23S and Q23E.

For example, compared to the amino acid sequence set forth in SEQ ID NO: 2, the amino acid sequence of the human GLP-1 polypeptide variant may comprise at least 2 amino acid mutations selected from the group consisting of: 1) K26R, 2) any one ofW31Y, W31R, W31K and W31A, and 3) any one of Y19A, Y19L, Y19T, Y19F, Y19I, Y19V and Y19S.

For example, compared to the amino acid sequence set forth in SEQ ID NO: 2, the amino acid sequence of the human GLP-1 polypeptide variant may comprise at least 2 amino acid mutations selected from the group consisting of: 1) K26R, 2) any one ofW31Y, W31R, W31K and W31A, and 3) any one of Q23N, Q23T, Q23S and Q23E.

In the present application, the amino acid sequence of the human GLP-1 polypeptide variant may comprise 2 amino acid substitutions at amino acid positions W31 and K26 compared to the amino acid sequence set forth in SEQ ID NO: 2, wherein the amino acid substitution at the position K26 may be K26R, and the amino acid substitution at the position W31 may be W31Y, W31R, W31K and W31A. For example, the amino acid sequence of the human GLP-1 polypeptide variant may comprise amino acid substitutions W31Y and K26R compared to the amino acid sequence set forth in SEQ ID NO: 2.

In the present application, the amino acid sequence of the human GLP-1 polypeptide variant may comprise amino acid substitution W31Y compared to the amino acid sequence set forth in SEQ ID NO: 2.

In the present application, the amino acid sequence of the human GLP-1 polypeptide variant may comprise amino acid substitution K26R compared to the amino acid sequence set forth in SEQ ID NO: 2.

In the present application, the amino acid sequence of the human GLP-1 polypeptide variant may comprise amino acid substitution Q23E compared to the amino acid sequence set forth in SEQ ID NO: 2.

In the present application, the amino acid sequence of the human GLP-1 polypeptide variant may comprise amino acid substitution Y19A compared to the amino acid sequence set forth in SEQ ID NO: 2.

In the present application, the amino acid sequence of the human GLP-1 polypeptide variant may comprise amino acid substitutions W31Y and Q23E compared to the amino acid sequence set forth in SEQ ID NO: 2.

In the present application, the amino acid sequence of the human GLP-1 polypeptide variant may comprise amino acid substitutions W31Y and Y19A compared to the amino acid sequence set forth in SEQ ID NO: 2.

In the present application, the amino acid sequence of the human GLP-1 polypeptide variant may comprise amino acid substitutions W31Y and K26R compared to the amino acid sequence set forth in SEQ ID NO: 2. For example, the human GLP-1 polypeptide variant may comprise an amino acid sequence set forth in any one of SEQ ID NOs: 29, 31 and 61-71. As another example, the human GLP-1 polypeptide variant may comprise an amino acid sequence set forth in any one of SEQ ID NOs: 29 and 61-71.

For example, the amino acid substitutions in the amino acid sequence of the human GLP-1 polypeptide variant may be W31Y and K26R. For example, the human GLP-1 polypeptide variant may comprise an amino acid sequence set forth in SEQ ID NO: 29.

In the present application, the amino acid sequence of the human GLP-1 polypeptide variant may comprise amino acid substitutions Q23E and K26R compared to the amino acid sequence set forth in SEQ ID NO: 2.

In the present application, the amino acid sequence of the human GLP-1 polypeptide variant may comprise amino acid substitutions Y19A and K26R compared to the amino acid sequence set forth in SEQ ID NO: 2.

In the present application, the amino acid sequence of the human GLP-1 polypeptide variant may comprise amino acid substitution K26R, an amino acid substitution at position W31, and an amino acid substitution at position Q23 compared to the amino acid sequence set forth in SEQ ID NO: 2. In the present application, the amino acid sequence of the human GLP-1 polypeptide variant may comprise amino acid substitution K26R, amino acid substitution W31Y, and an amino acid substitution at position Q23 compared to the amino acid sequence set forth in SEQ ID NO: 2. For example, the GLP-1 polypeptide variant may comprise an amino acid sequence set forth in any one of SEQ ID NOs: 61-64.

In the present application, the amino acid sequence of the human GLP-1 polypeptide variant may comprise amino acid substitution K26R, amino acid substitution W31Y, and an amino acid substitution at position Y19 compared to the amino acid sequence set forth in SEQ ID NO: 2. For example, the GLP-1 polypeptide variant may comprise an amino acid sequence set forth in any one of SEQ ID NOs: 65-71.

In the present application, the amino acid sequence of the human GLP-1 polypeptide variant may comprise amino acid substitution K26R, amino acid substitution W31Y, and amino acid substitution Q23N compared to the amino acid sequence set forth in SEQ ID NO: 2. For example, the GLP-1 polypeptide variant may comprise an amino acid sequence set forth in SEQ ID NO: 61.

In the present application, the amino acid sequence of the human GLP-1 polypeptide variant may comprise amino acid substitution K26R, amino acid substitution W31Y, and amino acid substitution Q23T compared to the amino acid sequence set forth in SEQ ID NO: 2. For example, the GLP-1 polypeptide variant may comprise an amino acid sequence set forth in SEQ ID NO: 62.

In the present application, the amino acid sequence of the human GLP-1 polypeptide variant may comprise amino acid substitution K26R, amino acid substitution W31Y, and amino acid substitution Q23S compared to the amino acid sequence set forth in SEQ ID NO: 2. For example, the GLP-1 polypeptide variant may comprise an amino acid sequence set forth in SEQ ID NO: 63.

In the present application, the amino acid sequence of the human GLP-1 polypeptide variant may comprise amino acid substitution K26R, amino acid substitution W31Y, and amino acid substitution Q23E compared to the amino acid sequence set forth in SEQ ID NO: 2. For example, the human GLP-1 polypeptide variant may comprise an amino acid sequence set forth in SEQ ID NO: 64.

In the present application, the amino acid sequence of the human GLP-1 polypeptide variant may comprise amino acid substitution K26R, amino acid substitution W31Y, and amino acid substitution Y19A compared to the amino acid sequence set forth in SEQ ID NO: 2. For example, the human GLP-1 polypeptide variant may comprise an amino acid sequence set forth in SEQ ID NO: 71.

In the present application, the amino acid sequence of the human GLP-1 polypeptide variant may comprise amino acid substitution K26R, amino acid substitution W31Y, and amino acid substitution Y19L compared to the amino acid sequence set forth in SEQ ID NO: 2. For example, the GLP-1 polypeptide variant may comprise an amino acid sequence set forth in SEQ ID NO: 66.

In the present application, the amino acid sequence of the human GLP-1 polypeptide variant may comprise amino acid substitution K26R, amino acid substitution W31Y, and amino acid substitution Y19T compared to the amino acid sequence set forth in SEQ ID NO: 2. For example, the GLP-1 polypeptide variant may comprise an amino acid sequence set forth in SEQ ID NO: 67.

In the present application, the amino acid sequence of the human GLP-1 polypeptide variant may comprise amino acid substitution K26R, amino acid substitution W31Y, and amino acid substitution Y19F compared to the amino acid sequence set forth in SEQ ID NO: 2. For example, the GLP-1 polypeptide variant may comprise an amino acid sequence set forth in SEQ ID NO: 65.

In the present application, the amino acid sequence of the human GLP-1 polypeptide variant may comprise amino acid substitution K26R, amino acid substitution W31Y, and amino acid substitution Y19I compared to the amino acid sequence set forth in SEQ ID NO: 2. For example, the GLP-1 polypeptide variant may comprise an amino acid sequence set forth in SEQ ID NO: 68.

In the present application, the amino acid sequence of the human GLP-1 polypeptide variant may comprise amino acid substitution K26R, amino acid substitution W31Y, and amino acid substitution Y19V compared to the amino acid sequence set forth in SEQ ID NO: 2. For example, the GLP-1 polypeptide variant may comprise an amino acid sequence set forth in SEQ ID NO: 69.

In the present application, the amino acid sequence of the human GLP-1 polypeptide variant may comprise amino acid substitution K26R, amino acid substitution W31Y, and amino acid substitution Y19S compared to the amino acid sequence set forth in SEQ ID NO: 2. For example, the GLP-1 polypeptide variant may comprise an amino acid sequence set forth in SEQ ID NO: 70.

In the present application, the human GLP-1 polypeptide variant may comprise an amino acid sequence set forth in SEQ ID NO: 153: HGEGTFTSDVSSX₁₉LEEX₂₃AAX₂₆EFIAX₃₁LVKGGG, where X₁₉ = Y, A, L, T, F, I, V or S; X₂₃ = Q, E, T, S or N; X₂₆ = K or R; X₃₁ = W, A, R, K or Y. X₁₉ = Y, X₂₃ = Q, X₂₆ = K and X₃₁ = W are not present at the same time, or at least three of X₁₉ = Y, X₂₃ = Q, X₂₆ = K and X₃₁ = W are not present at the same time.

In the present application, compared to the amino acid sequence set forth in SEQ ID NO: 2, the human GLP-1 polypeptide variant may comprise an amino acid sequence set forth in SEQ ID NO: 153: HGEGTFTSDVSSX₁₉LEEX₂₃AAX₂₆EFIAX₃₁LVKGGG, and may comprise at least 2 amino acid substitutions at positions X₁₉, X₂₃, X₂₆ and/or X₃₁, wherein amino acid Y at position X₁₉ may be substituted with A, L, T, F, I, V or S; amino acid Q at position X₂₃ may be substituted with E, T, S or N; amino acid K at position X₂₆ may be substituted with R; amino acid W at position X₃₁ may be substituted with Y, K, R or A.

In the present application, the human GLP-1 polypeptide variant may comprise an amino acid sequence set forth in SEQ ID NO: 150: HGEGTFTSDVSSX₁₉LEEX₂₃AAREFIAYLVKGGG, where X₁₉ = Y, A, L, T, F, I, V or S; X₂₃ = Q, E, T, S or N. X₁₉ = Y and X₂₃ = Q are not present at the same time. In the present application, compared to the amino acid sequence set forth in SEQ ID NO: 2, the human GLP-1 polypeptide variant may comprise an amino acid sequence set forth in SEQ ID NO: 150: HGEGTFTSDVSSX₁₉LEEX₂₃AAREFIAYLVKGGG, and may comprise amino acid substitutions at positions X₁₉ and X₂₃, wherein amino acid Y at position X₁₉ may be substituted with A, L, T, F, I, V or S; amino acid Q at position X₂₃ may be substituted with E, T, S or N.

In the present application, the amino acid sequence of the human GLP-1 polypeptide variant may comprise an amino acid sequence set forth in SEQ ID NO: 151: HGEGTFTSDVSSYLEEX₂₃AAREFIAYLVKGGG, where X₂₃ = E, T, S or N. In the present application, the amino acid sequence of the human GLP-1 polypeptide variant may comprise an amino acid sequence set forth in SEQ ID NO: 151; compared to the amino acid sequence set forth in SEQ ID NO: 2, the amino acid sequence of the human GLP-1 polypeptide variant may comprise an amino acid substitution at position X₂₃, wherein amino acid Q at position X₂₃ may be substituted with E, T, S or N.

In the present application, the amino acid sequence of the human GLP-1 polypeptide variant may comprise an amino acid sequence set forth in SEQ ID NO: 152: HGEGTFTSDVSSX₁₉LEEQAAREFIAYLVKGGG, where X₁₉ = A, L, T, F, I, V or S. In the present application, the amino acid sequence of the human GLP-1 polypeptide variant may comprise an amino acid sequence set forth in SEQ ID NO: 151; compared to the amino acid sequence set forth in SEQ ID NO: 2, the amino acid sequence of the human GLP-1 polypeptide variant may comprise an amino acid substitution at position X₁₉, wherein amino acid Y at position X₁₉ may be substituted with A, L, T, F, I, V or S.

### Fusion Protein or Immunoconjugate

In another aspect, the present application provides a fusion protein or immunoconjugate which may comprise the human GLP-1 polypeptide variant described herein. In the present application, the fusion protein or immunoconjugate may comprise 1, 2 or more (e.g., 3, 4 or 5) of the human GLP-1 polypeptide variants.

In the present application, the fusion protein or immunoconjugate may further comprise an immunoglobulin Fc region. The immunoglobulin Fc region described herein may include the heavy chain constant region 2 (CH₂) and heavy chain constant region 3 (CH₃) of an immunoglobulin, and typically does not comprise two antigen-binding regions (Fab fragments) from the antibody. In addition, the immunoglobulin Fc region of the present application may comprise the entirety or part of the Fc region, so long as it has similar physiological activity to the native protein. The Fc region of the present application may include Fc regions from IgA, IgD, IgE, IgG and IgM. For example, the Fc region may be an Fc region derived from IgG. The Fc region may be derived from human IgG. For example, the Fc region may be an Fc region derived from IgG1, IgG2, IgG3 or IgG4. For example, the Fc region may be derived from human IgG4.

In the present application, the Fc region of the fusion protein or immunoconjugate may further comprise an immunoglobulin hinge region. The hinge region may be derived from IgG. The hinge region may be derived from human IgG. For example, the hinge region may be a hinge region derived from IgG1, IgG2, IgG3 or IgG4. For example, the hinge region may be located at the C-terminus of the GLP-1 polypeptide variant. For example, the hinge region may be located at the N-terminus of the Fc region CH₂.

For example, the Fc region may comprise an amino acid sequence set forth in SEQ ID NOs: 139-143. For example, the Fc region of the fusion protein or immunoconjugate may comprise an amino acid sequence set forth in SEQ ID NO: 142 or 143. As another example, the Fc region may comprise an amino acid sequence set forth in SEQ ID NO: 143.

In the present application, one end of the GLP-1 polypeptide variant may be linked directly or indirectly to one end of the Fc region, for example, via in-frame fusion. In certain cases, the C-terminus of the GLP-1 polypeptide variant may be linked directly or indirectly to the N-terminus of the Fc region.

In the present application, the fusion protein or immunoconjugate may comprise the GLP-1 polypeptide variant and an immunoglobulin Fc region. In certain cases, the GLP-1 polypeptide variant and the Fc may be linked by a linker (e.g., a linker peptide). For example, the linker peptide may be selected from GS, GAP, G4S, (G4S)2, (G4S)3 and (G4S)4. For example, the linker peptide may comprise an amino acid sequence set forth in any one of SEQ ID NOs: 144-149.

In the present application, the fusion protein or immunoconjugate may comprise, in order from N-terminus to C-terminus, any one of the GLP-1 polypeptide variants described herein, the linker peptide, and the Fc region. The fusion protein or immunoconjugate may comprise, in order from N-terminus to C-terminus, any one of the GLP-1 polypeptide variants described herein, the linker peptide (G4S)3 (of which the amino acid sequence is set forth in SEQ ID NO: 148), and an Fc region derived from IgG4 (of which the amino acid sequence is set forth in SEQ ID NO: 143). For example, the fusion protein or immunoconjugate described herein may comprise an amino acid sequence set forth in any one of SEQ ID NOs: 96, 98-120 and 128-138.

The fusion protein or immunoconjugate described herein has biological activity. Biological activity refers to the ability to bind, and activate GLP-1R, and elicit responses *in vivo* or *in vitro.* The responses include, but are not limited to, secretion of insulin, suppression of glucagon, suppression of appetite, weight loss, induction of satiety, suppression of apoptosis, and induction of pancreatic β cell proliferation and pancreatic β cell differentiation. Some representative fusion proteins are tested for *in vitro* and *in vivo* activity.

The fusion protein or immunoconjugate described herein reflects the ability of the fusion protein of the present application to interact with human GLP-1R, for example, as measured by ELISA, in Example 2. Example 3 reflects the ability of the fusion protein to activate GLP-1R *in vitro.* GLP-1R activation leads to adenylate cyclase activation, which induces the expression of the cAMP response element binding protein (CREB) driven reporter gene, for example, as reflected by the fluorescence intensity of the luciferase reporter gene, or the level of cAMP expression. Example 4 reflects that the fusion protein has a relatively long blood half-life. Example 5 reflects the ability of the fusion protein described herein to reduce elevated blood sugar levels.

In the present application, the human GLP-1 polypeptide variant is designated GLP-1-XX, where "XX" represents an amino acid substitution. For example, for the human GLP-1 polypeptide variant GLP-WY, it represents that compared to the amino acid sequence set forth in SEQ ID NO: 2, the amino acids of the human GLP-1 polypeptide variant GLP-WY are mutated as follows: amino acid residue W at position 31 is substituted with amino acid residue Y; for the human GLP-1 polypeptide variant GLP-KRWYYA, it represents that compared to the amino acid sequence set forth in SEQ ID NO: 2, the amino acids of the human GLP-1 polypeptide variant GLP-KRWYYA are mutated as follows: amino acid residue Y at position 19 is substituted with amino acid residue A, amino acid residue K at position 26 is substituted with amino acid residue R, and amino acid residue W at position 31 is substituted with amino acid residue Y.

In the present application, the fusion protein of the human GLP-1 polypeptide variant and the Fc region is designated GM-XX, where "XX" represents a corresponding amino acid substitution in the human GLP-1 polypeptide variant.

### Nucleic Acid Molecule, Vector, Cell and Preparation Method

In another aspect, the present application also provides one or more isolated nucleic acid molecules capable of encoding the polypeptide variant or the fusion protein or immunoconjugate described herein. For example, each of the one or more nucleic acid molecules is capable of encoding the entirety or part of the polypeptide variant or the fusion protein or immunoconjugate.

The nucleic acid molecules described herein may be isolated. For example, it may be produced or synthesized by the following methods: (i) *in vitro* amplification, such as amplification by polymerase chain reaction (PCR); (ii) cloning and recombination; (iii) purification, such as separation by enzymatic digestion and gel electrophoresis fractionation; or (iv) synthesis, such as chemical synthesis. In certain embodiments, the isolated nucleic acid is a nucleic acid molecule prepared by recombinant DNA techniques. Recombinant DNA and molecular cloning techniques include those described in Sambrook, J., Fritsch, E. F. and Maniatis, T. Molecular Cloning: A Laboratory Manual; Cold Spring Harbor Laboratory Press: Cold Spring Harbor, (1989) (Maniatis) and T. J. Silhavy, M. L. Bennan and L. W. Enquist, Experiments with Gene Fusions, Cold Spring Harbor Laboratory, Cold Spring Harbor, N.Y. (1984), as well as Ausubel, F. M. et al., Current Protocols in Molecular Biology, pub. by Greene Publishing Assoc. and Wiley-Interscience (1987). Briefly, the nucleic acids can be prepared from genomic DNA fragments, cDNA and RNA, and all of the nucleic acids can be directly extracted from cells or recombinantly produced by various amplification methods, including but not limited to PCR and RT-PCR.

In another aspect, the present application provides one or more vectors comprising the nucleic acid molecules. For example, the vector may comprise one or more of the nucleic acid molecules. In addition, the vector may further comprise other genes, such as marker genes that allow selection of the vector in an appropriate host cell and under appropriate conditions. In addition, the vector may further comprise expression control elements that allow proper expression of the coding region in an appropriate host. Such control elements are well known to those skilled in the art and may include, for example, promoters, ribosome binding sites, enhancers and other control elements for regulating gene transcription or mRNA translation. The one or more nucleic acid molecules described herein may be operably linked to the expression control elements.

The vector may include, for example, plasmids, cosmids, viruses, phages or other vectors commonly used in, for example, genetic engineering. For example, the vector is an expression vector.

In another aspect, the present application provides a cell that may comprise the one or more nucleic acid molecules described herein and/or the one or more vectors described herein. In certain embodiments, each type of or each host cell may comprise one type of or one nucleic acid molecule or vector described herein. In certain embodiments, each type of or each host cell may comprise multiple (e.g., two or more) or multiple types (e.g., two or more) of nucleic acid molecules or vectors described herein. For example, the vector described herein can be introduced into the cell, e.g., a prokaryotic cell (e.g., a bacterial cell), a CHO cell, an NS0 cell, an HEK293T cell or an HEK293A cell, or other eukaryotic cells, such as a plant-derived cell or a fungal or yeast cell. The vector described herein can be introduced into the cell by methods known in the art, such as electroporation and liposomal transfection.

In another aspect, the present application provides a method for preparing the polypeptide variant or the fusion protein or immunoconjugate described herein. The method may comprise culturing the cell described herein under conditions that allow expression of the polypeptide variant or the fusion protein or immunoconjugate, for example, by adopting an appropriate culture medium, an appropriate temperature, an appropriate culture time and the like. These methods are known to those of ordinary skill in the art.

In certain cases, the method may further comprise the step of harvesting (e.g., isolating and/or purifying) the polypeptide variant or the fusion protein or immunoconjugate described herein. For example, affinity chromatography can be performed with protein G-agarose or protein A-agarose; the polypeptide variant or the fusion protein or immunoconjugate described herein can also be purified and isolated by gel electrophoresis and/or high performance liquid chromatography, etc.

### Pharmaceutical Composition and Use

In another aspect, the present application provides a pharmaceutical composition comprising the human GLP-1 polypeptide variant, the fusion protein or immunoconjugate, the nucleic acid molecule, the vector, or the cell, and optionally a pharmaceutically acceptable adjuvant.

For example, the pharmaceutically acceptable adjuvant may include buffer, antioxidant, preservative, low molecular weight polypeptide, protein, hydrophilic polymer, amino acid, sugar, chelating agent, counter ion, metal complex, and/or nonionic surfactant, and the like.

In the present application, the pharmaceutical composition may be formulated for oral administration, intravenous administration, intramuscular administration, *in situ* administration at the tumor site, inhalation, rectal administration, vaginal administration, transdermal administration or administration via a subcutaneous depot.

The pharmaceutical composition described herein can be used to treat metabolic diseases or conditions. The pharmaceutical composition can be used to treat diabetes (e.g. hyperglycemia, type I diabetes, type II diabetes, impaired glucose tolerance, non-insulin-dependent diabetes, MODY (maturity-onset diabetes of the young), gestational diabetes and/or to lower glycated hemoglobin (HbAlC)), prediabetes, hypertension, hyperglycemia and/or dyslipidemia (or combinations of these metabolic risk factors). Acute symptoms of diabetes include excessive urine production, resulting compensatory thirst and increased fluid intake, blurred vision, unexplained weight loss, hypersomnia, and altered energy metabolism. The chronic hyperglycemia of diabetes is associated with macrovascular and microvascular complications that may lead to long-term damage to, impairment in the function of and, in some cases, eventual failure of various organs, especially the eyes (especially in the form of diabetic retinopathy), the kidney (in the form of diabetic nephropathy), nerves (in the form of diabetic neuropathy), the heart, and blood vessels. The pharmaceutical compositions can also be used to treat weight gain, promote weight loss, reduce excess weight and/or treat obesity (e.g., by controlling appetite, eating, food intake, calorie intake and/or energy expenditure), including morbid obesity, and related diseases, abnormalities and health conditions, including but not limited to obesity-related inflammation, obesity-related gallbladder diseases, and obesity-induced sleep apnea. For example, the metabolic diseases or conditions may be selected from diabetes, obesity and other GLP-1-associated metabolic conditions.

The frequency of administration and the dose of the pharmaceutical composition can be determined by a variety of relevant factors, including the type of the disease to be treated, the route of administration, the age, sex and body weight of the patient and the severity of the disease, as well as the type of the drug used as an active ingredient. Since the pharmaceutical composition has excellent durations of *in vivo* efficacy and concentration, it can significantly reduce the frequency of administration and the dose of the drug.

Without being limited by any theory, the following examples are intended only to illustrate the fusion protein, preparation method, use, etc., of the present application, and are not intended to limit the scope of the present application.

### Examples

The positive control dulaglutide used in the examples of the present application is a diabetes drug of Eli Lilly & Co under the code number LY-2189265. The amino acid sequence of dulaglutide is set forth in SEQ ID NO: 4. Dulaglutide is referred to herein as GLP-1-Fc or KN042.

### Example 1. Preparation of Fusion Proteins

Fusion proteins of GLP-1 polypeptide variants GLP-1-XX and Fc were prepared. The fusion proteins comprise, in order from N-terminus to C-terminus, a GLP-1 polypeptide variant (of which the amino acid sequence is set forth in any one of SEQ ID NOs: 5-71), the linker peptide (G4S)3, and an Fc region derived from IgG4.

Vectors comprising nucleic acid sequences encoding the fusion proteins were transfected into cells, and the fusion proteins GM-XX were expressed and purified. The amino acid sequence is set forth in any one of SEQ ID NOs: 72-138. The amino acid mutations and sequences of the fusion proteins of the present application and the corresponding human GLP-1 polypeptide variants are shown in Table 1.

**Table 1. The amino acid mutations and sequences of the fusion proteins and human GLP-1 polypeptide variants used in the examples**

| Fusion protein | Human GLP-1 polypeptide variant | | |
|---|---|---|---|
| | Name | Amino acid mutation(s) | SEQ ID NO |
| GM-WY | GLP-1-WY | W31Y | 5 |
| GM-WR | GLP-1-WR | W31R | 6 |
| GM-WK | GLP-1-WK | W31K | 7 |
| GM-FY | GLP-1-FY | F28Y | 8 |
| GM-FH | GLP-1-FH | F28H | 9 |
| GM-FT | GLP-1-FT | F28T | 10 |
| GM-FN | GLP-1-FN | F28N | 11 |
| GM-FS | GLP-1-FS | F28S | 12 |
| GM-FQ | GLP-1-FQ | F28Q | 13 |
| GM-FE | GLP-1-FE | F28E | 14 |
| GM-FV | GLP-1-FV | F28V | 15 |
| GM-FK | GLP-1-FK | F28K | 16 |
| GM-FI | GLP-1-FI | F28I | 17 |
| GM-QN | GLP-1-QN | Q23N | 18 |
| GM-QT | GLP-1-QT | Q23T | 19 |
| GM-QS | GLP-1-QS | Q23S | 20 |
| GM-QE | GLP-1-QE | Q23E | 21 |
| GM-YL | GLP-1-YL | Y19L | 22 |
| GM-YT | GLP-1-YT | Y19T | 23 |
| GM-YF | GLP-1-YF | Y19F | 24 |
| GM-YI | GLP-1-YI | Y19I | 25 |
| GM-YV | GLP-1-YV | Y19V | 26 |
| GM-YS | GLP-1-YS | Y19S | 27 |
| GM-YA | GLP-1-YA | Y19A | 28 |
| GM-KRWY | GLP-1-KRWY | W31Y, K26R | 29 |
| GM-KRFY | GLP-1-KRFY | F28Y, K26R | 30 |
| GM-KRWYFY | GLP-1-KRWYFY | W31Y, K26R, F28Y | 31 |
| GM-KRQN | GLP-1-KRQN | K26R, Q23N | 32 |
| GM-KRQT | GLP-1-KRQT | K26R, Q23T | 33 |
| GM-KRQS | GLP-1-KRQS | K26R, Q23S | 34 |
| GM-KRQE | GLP-1-KRQE | K26R, Q23E | 35 |
| GM-KRYL | GLP-1-KRYL | K26R, Y19L | 36 |
| GM-KRYT | GLP-1-KRYT | K26R, Y19T | 37 |
| GM-KRYF | GLP-1-KRYF | K26R, Y19F | 38 |
| GM-KRYI | GLP-1-KRYI | K26R, Y19I | 39 |
| GM-KRYV | GLP-1-KRYV | K26R, Y19V | 40 |
| GM-KRYS | GLP-1-KRYS | K26R, Y19S | 41 |
| GM-KRYA | GLP-1-KRYA | K26R, Y19A | 42 |
| GM-WYQN | GLP-1-WYQN | W31Y, Q23N | 43 |
| GM-WYQS | GLP-1-WYQS | W31Y, Q23S | 44 |
| GM-WYQT | GLP-1-WYQT | W31Y, Q23T | 45 |
| GM-WYQE | GLP-1-WYQE | W31Y, Q23E | 46 |
| GM-WYYL | GLP-1-WYYL | W31Y, Y19L | 47 |
| GM-WYYT | GLP-1-WYYT | W31Y, Y19T | 48 |
| GM-WYYF | GLP-1-WYYF | W31Y, Y19F | 49 |
| GM-WYYI | GLP-1-WYYI | W31Y, Y19I | 50 |
| GM-WYYV | GLP-1-WYYV | W31Y, Y19V | 51 |
| GM-WYYS | GLP-1-WYYS | W31Y, Y19S | 52 |
| GM-WYYA | GLP-1-WYYA | W31Y, Y19A | 53 |
| GM-WYFY | GLP-1-WYFY | W31Y, F28Y | 54 |
| GM-WYFL | GLP-1-WYFL | W31Y, F28L | 55 |
| GM-WA | GLP-1-WA | W31A | 56 |
| GM-WL | GLP-1-WL | W31L | 57 |
| GM-FL | GLP-1-FL | F28L | 58 |
| GM-WIFL | GLP-1-WIFL | W31I, F28L | 59 |
| GM-WLFL | GLP-1-WLFL | W31L, F28L | 60 |
| GM-KRWYQN | GLP-1-KRWYQN | W31Y, K26R, Q23N | 61 |
| GM-KRWYQT | GLP-1-KRWYQT | W31Y, K26R, Q23T | 62 |
| GM-KRWYQS | GLP-1-KRWYQS | W31Y, K26R, Q23S | 63 |
| GM-KRWYQE | GLP-1-KRWYQE | W31Y, K26R, Q23E | 64 |
| GM-KRWYYF | GLP-1-KRWYYF | W31Y, K26R, Y19F | 65 |
| GM-KRWYYL | GLP-1-KRWYYL | W31Y, K26R, Y19L | 66 |
| GM-KRWYYT | GLP-1-KRWYYT | W31Y, K26R, Y19T | 67 |
| GM-KRWYYI | GLP-1-KRWYYI | W31Y, K26R, Y19I | 68 |
| GM-KRWYYV | GLP-1-KRWYYV | W31Y, K26R, Y19V | 69 |
| GM-KRWYYS | GLP-1-KRWYYS | W31Y, K26R, Y19S | 70 |
| GM-KRWYYA | GLP-1-KRWYYA | W31Y, K26R, Y19A | 71 |
| GM-KAWA | GLP-1-KAWA | W31A, K26A | 154 |

### Example 2. Binding Affinities of the Fusion Proteins of the Present Application for GLP-1 Receptor

The binding affinities of the fusion proteins of Example 1 for the GLP-1 receptor were measured by ELISA. A fusion protein of the GLP-1 receptor and mouse Fc was expressed, and the fusion protein was referred to as GLP1R-muFc (SEQ ID NO: 3). GLP1R-muFc was diluted to 5 µg/mL and used to coat a 96-well plate; it was fixed at room temperature for 30 min. The plate was washed with PBS once, then blocked with 1% BSA at 37 °C for 30 min, and washed with 0.05% PBST20 three times. Test samples (4-fold serial dilutions, 10 µg/mL) were added at 50 µL/well. KN042 was used as a positive control. The plate was incubated at 37 °C for 1 h and washed with PBST five times. Mouse monoclonal HP6023 anti-human IgG4Fc-HRP (abcam, ab99817, 2000-fold diluted with 0.05% PBST20 containing 1% BSA) was added at 50 µL/well. The plate was incubated at 37 °C for 1 h and washed with PBST five times. To each well was added 50 µL of TMB, and color development was allowed for 3 min. To each well was added 50 µL of 1 M H₂SO₄ to stop the reaction. The OD values were measured on a microplate reader (Molecular Devices, SpectraMax M3). KN042 was used as a positive control.

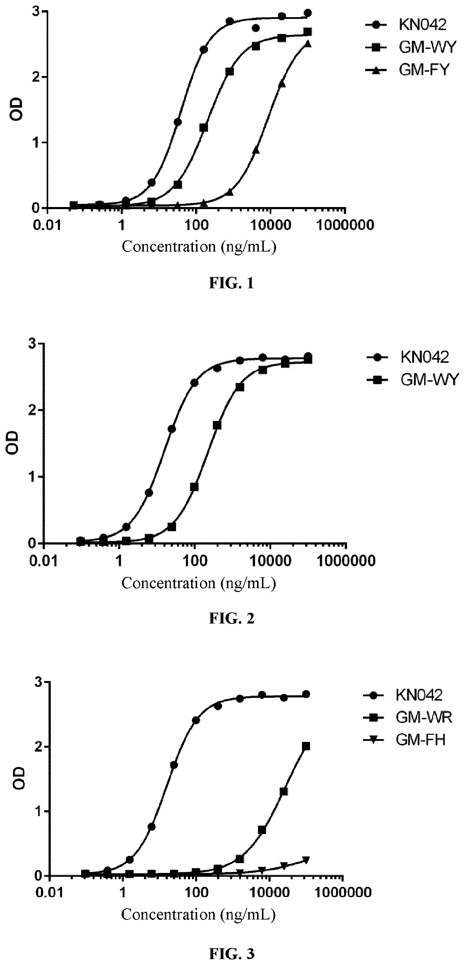

The results are shown in FIGs. 1-13, 16-20 and 33.

FIG. 1 shows that the fusion protein with amino acid mutation W31Y has better binding affinity for the GLP-1 receptor than the fusion protein with mutation F28Y.

FIG. 2 shows that the fusion protein with amino acid mutation W31Y is similar to the positive control KN042.

FIG. 3 shows that the fusion protein with an amino acid mutation at position W31 has better binding activity to the GLP-1 receptor than the fusion protein with an amino acid mutation at position F28.

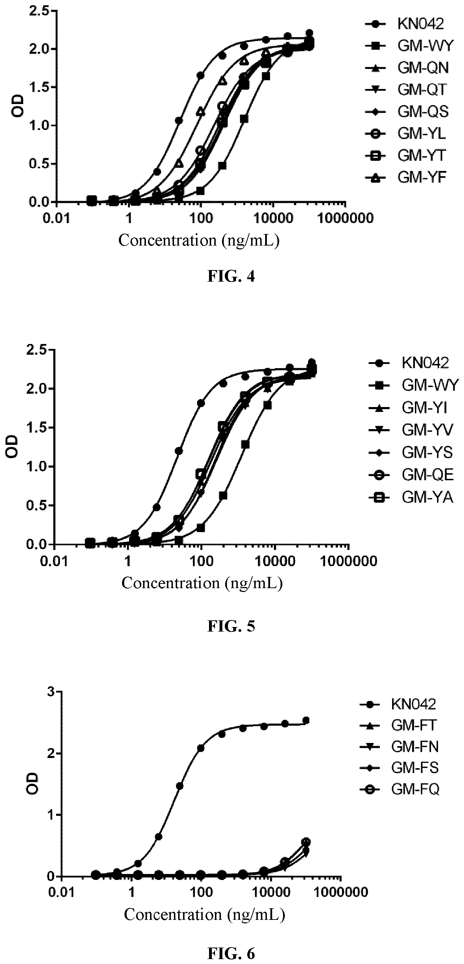

FIG. 4 and FIG. 5 show that both the fusion proteins with various amino acid mutations at position Q23 and the fusion proteins with various amino acid mutations at position Y19 retained the binding activity to the GLP-1 receptor.

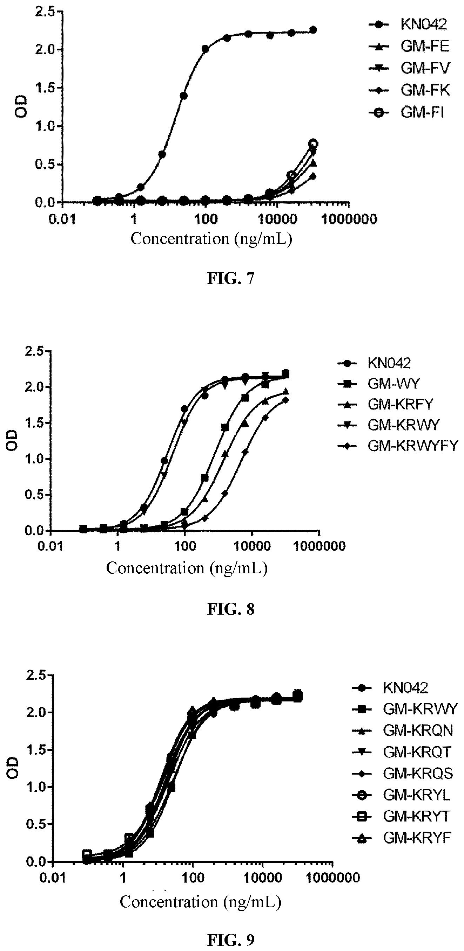

FIG. 6 and FIG. 7 show that the fusion proteins with an amino acid mutation at position F28 have significantly lower binding activity to the GLP-1 receptor.

As can be seen from FIG. 1 to FIG. 7, the fusion proteins with amino acid mutations at positions W31, Q23 and Y19 retained the binding activity to the GLP-1 receptor, while the amino acid mutation at position F28 caused a significant decrease in the binding activity to the GLP-1 receptor.

FIG. 8 shows that the fusion protein with double mutation K26R-W31Y has better binding activity to the GLP-1 receptor than the fusion protein with double mutation K26R-F28Y.

FIG. 9 shows that the fusion proteins with double mutations K26R-W31Y, K26R-Q23T, K26R-Q23S, K26R-Q23N, K26R-Y19L, K26R-Y19T and K26R-Y19F have good binding activity to the GLP-1 receptor, similar to the positive control KN042.

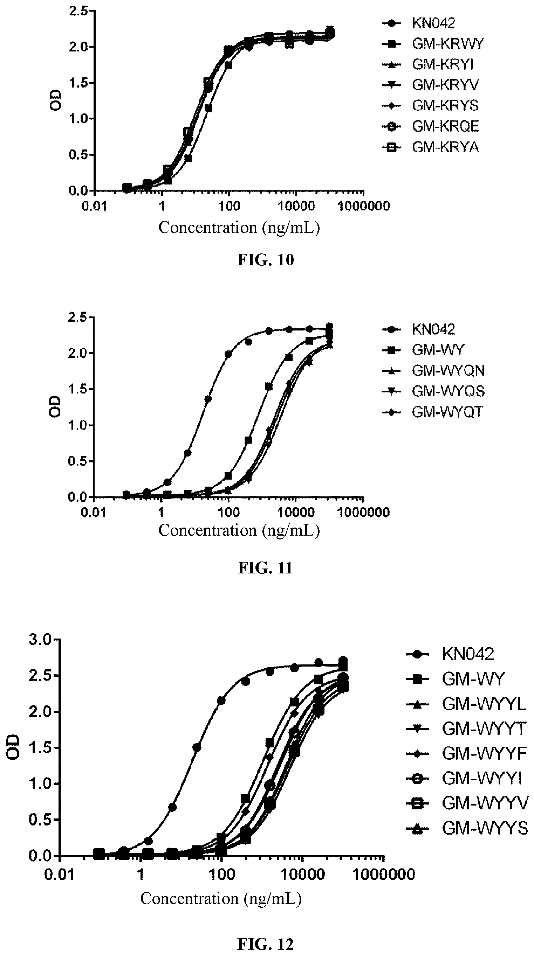

FIG. 10 shows that the fusion proteins with double mutations K26R-W31Y, K26R-Q23E, K26R-Y19I, K26R-Y19V, K26R-Y19A and K26R-Y19S have good binding activity to the GLP-1 receptor, similar to the positive control KN042.

FIG. 11 shows that the fusion proteins with double mutations K26R-W31Y, K26R-Q23N, K26R-Q23S and K26R-Q23T have good binding activity to the GLP-1 receptor.

FIG. 12 shows that the fusion proteins with double amino acid mutations W31Y-Y19 have good binding activity to the GLP-1 receptor.

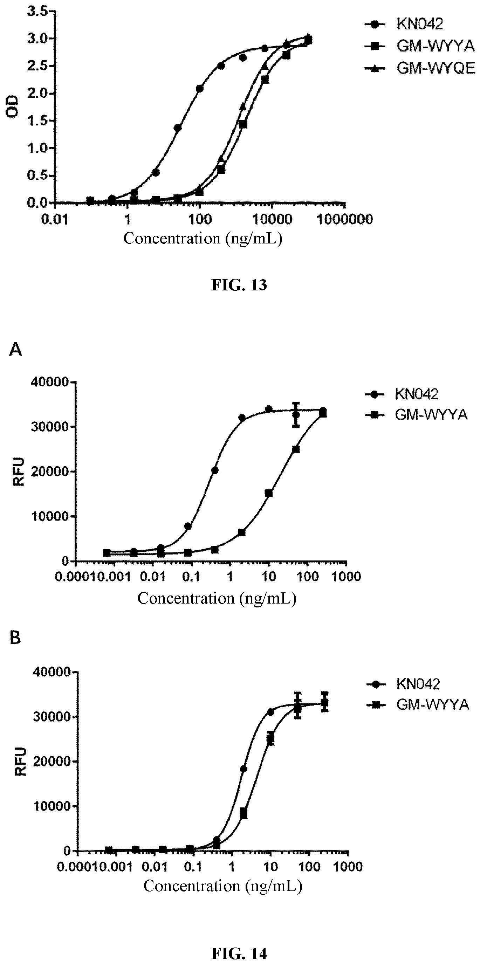

FIG. 13 shows that the fusion proteins with double amino acid mutations W31Y-Y19A and W31Y-Q23E have good binding activity to the GLP-1 receptor.

As can be seen from FIG. 8 to FIG. 13, the fusion proteins comprising amino acid mutations at 2 of positions K26, W31, Y19 and Q23 have good binding activity to the GLP-1 receptor.

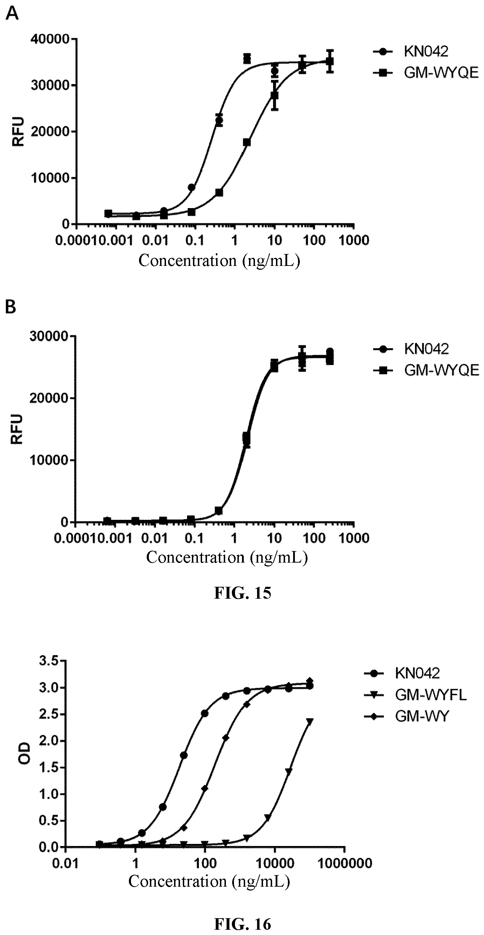

FIG. 16 shows that both GM-WY and GM-WYFL can bind to the GLP-1 receptor.

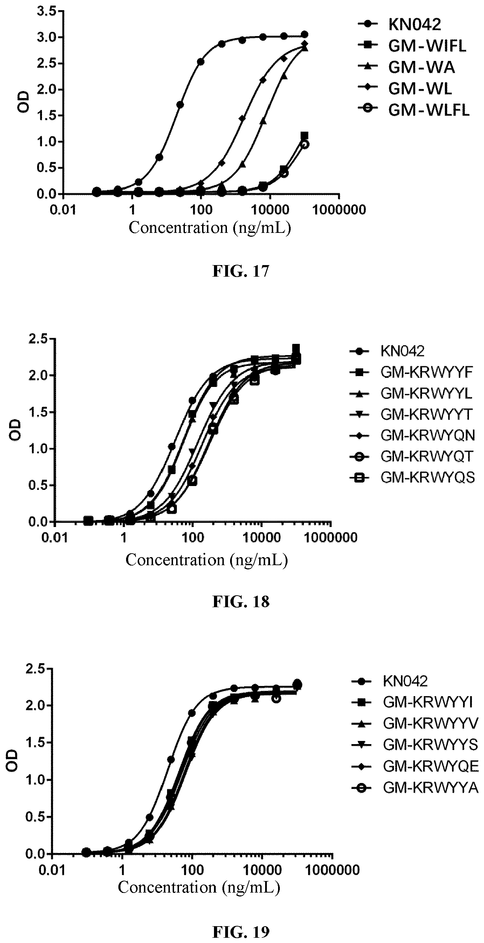

FIG. 17 shows that all of GM-WIFL, GM-WA, GM-WL and GM-WLFL can bind to the GLP-1 receptor.

FIGs. 16 and 17 show that the fusion proteins of the GLP-1 polypeptide variants that did not comprise amino acid mutations at at least 2 amino acid positions selected from W31, Y19, Q23 and K26 have relatively weak binding activity.

FIG. 18 shows that the fusion proteins with triple amino acid mutations W31Y-K26R-Q23 or W31Y-K26R-Y19 have good binding activity to the GLP-1 receptor.

FIG. 19 shows that the fusion proteins with triple amino acid mutations W31Y-K26R-Q23 or W31Y-K26R-Y19 have good binding activity to the GLP-1 receptor.

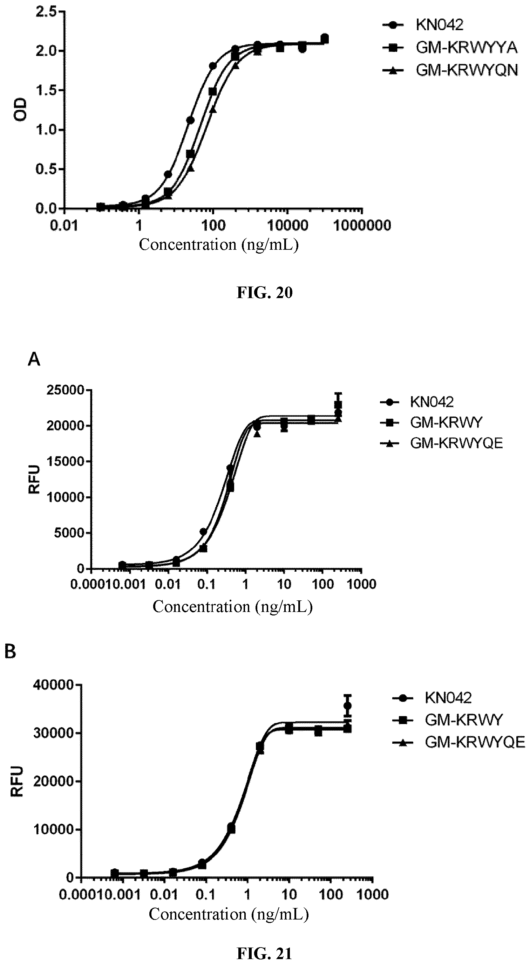

FIG. 20 shows that the fusion protein with triple amino acid mutation W31Y-K26R-Q23N or W31Y-K26R-Y19Ahas good binding activity to the GLP-1 receptor.

As can be seen from FIG. 18 to FIG. 20, the fusion proteins comprising amino acid mutations at 3 of positions K26, W31, Y19 and Q23 have good binding activity to the GLP-1 receptor.

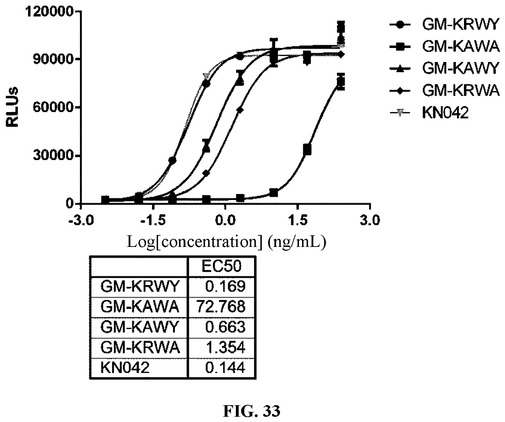

The experimental results of the present application also show that the fusion protein with double mutation K26R-W31Y has better *in vitro* biological activity (e.g., the binding activity to the GLP-1 receptor or the activity of activating the cAMP/PKA signaling pathway) than the fusion proteins with double mutations K26R-W31A and K26A-W31Y. For example, the results in FIG. 33 demonstrate that the fusion protein with double mutation K26R-W31Y has better binding activity to the GLP-1 receptor than the fusion proteins with double mutations K26R-W31A, K26A-W31Y and K26A-W31A.

### Example 3. Luciferase Assays for the Fusion Proteins of the Present Application's Effect of Activating cAMP/PKA Signaling Pathway

On the basis of the principle that fusion proteins of GLP-1 polypeptide variants binding to the GLP-1 receptor activates adenylate cyclase and promotes an increase in the intracellular cyclic adenosine monophosphate (cAMP) level, the fusion proteins of the present application were assayed for the effect of activating the cAMP/PKA signaling pathway using the CREB (cAMP response element binding protein) reporter gene. HER293 cells were transfected with the human GLP-1R plasmid and the CREB-driven luciferase reporter plasmid. After transfection, HER293-GLP1R-CREB-D4 cells were digested, harvested, and counted, and the cell density was adjusted to 4 × 10⁵ cells/mL in a DMEM medium containing 10% FBS. The cells were inoculated onto a 96-well plate at 50 µL/well. Test samples were diluted with DMEM medium containing 10% FBS from 1000 ng/mL to 9 concentrations; KN042 was used as a positive control. The various concentrations of test samples were then added to the above 96-well plate at 50 µL/well, and the plate was incubated at 37 °C for 6 h or 24 h. The supernatant was discarded and luciferase substrate was added to each well. After the plate was left to stand at room temperature for 5 min, 50 µL was taken and assayed for luciferase activity on Bio-Glo^{™} Luciferase Assay System (Promega, G7940), and readings were taken on a microplate reader (Molecular Devices, SpectraMax M3). The fluorescence intensity reflects the level of cAMP and further the degree of GLP-1 receptor activation.

The results are shown in FIGs. 14-15 and FIGs. 21-26. The fusion proteins of the present application can activate the GLP-1 receptor and thus the cAMP/PKA signaling pathway. The fusion proteins GM-WYYA, GM-WYQE, GM-KRWY, GM-KRWYQE, GM-KRWYYA, GM-KRWYYL, GM-KRWYYS, GM-KRWYYI, GM-KRWYYT, GM-KRWYQN, GM-KRWYQT and GM-KRWYQS were shown to have good *in vitro* stability.

FIG. 14 shows that the fusion protein of the GLP-1 polypeptide variant and the Fc region with double mutation W31Y-Y19A can activate the GLP-1 receptor, and thus the cAMP level was increased; in addition, the activating ability is stronger when the incubation time is extended (increased from 6 h (14A) to 24 h (14B)). The activating ability is better than that of the positive control, which indicates that the fusion protein GM-WYYA of the present application showed better *in vitro* stability.

FIG. 15 shows that the fusion protein of the GLP-1 polypeptide variant and the Fc region with double mutation W31Y-Q23E can activate the GLP-1 receptor, and thus the cAMP level was increased; in addition, the activating ability is stronger when the incubation time is extended (increased from 6 h (15A) to 24 h (15B)). The activating ability is better than that of the positive control, which indicates that the fusion protein GM-WYQE of the present application showed better *in vitro* stability.

FIG. 21 shows that both the fusion protein with double mutation K26R-W31Y and the fusion proteins with triple amino acid mutations K26R-W31Y-Q23 can activate the GLP-1 receptor.

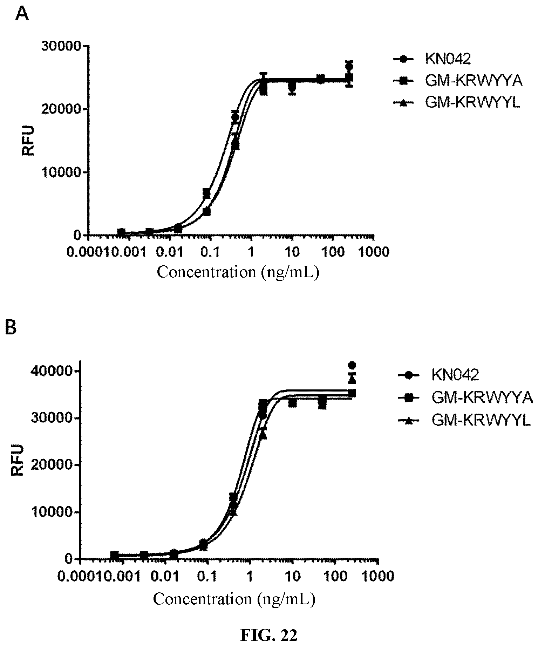

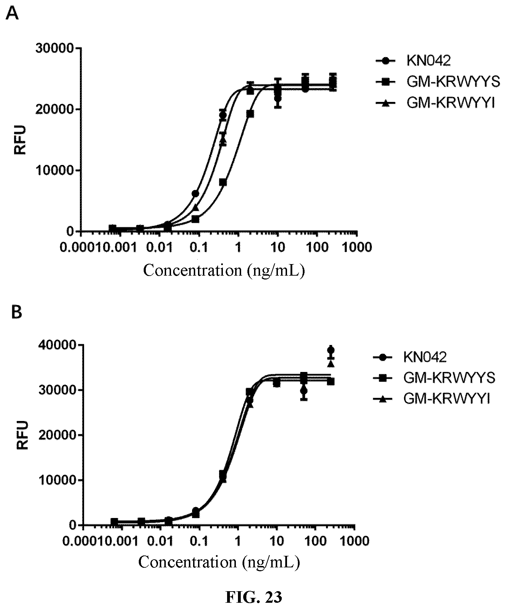

FIGs. 22 and 23 show that all the fusion proteins with triple amino acid mutations K26R-W31Y-Y19 can activate the GLP-1 receptor.

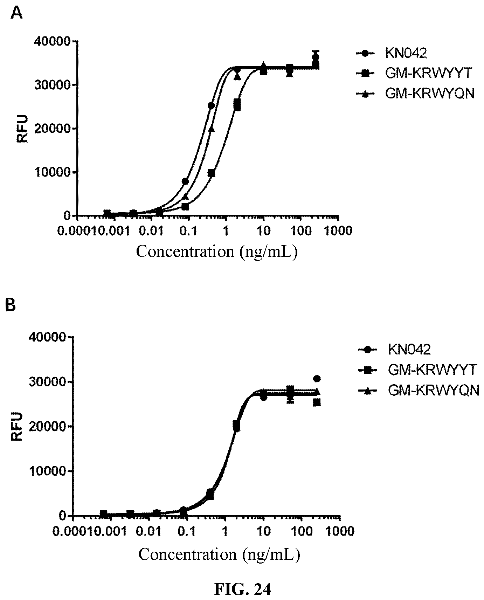

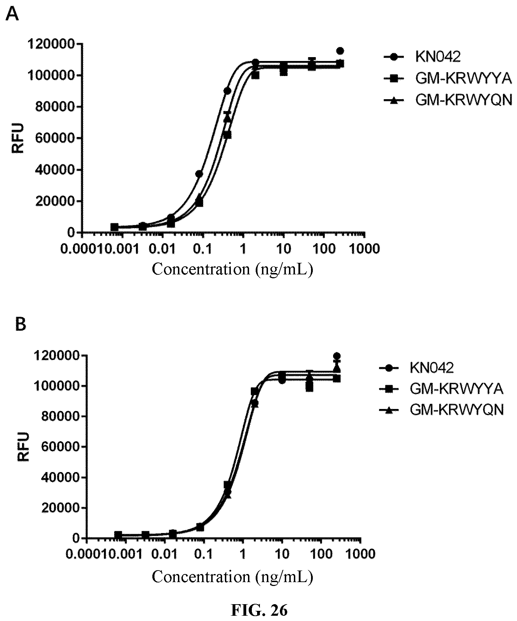

FIG. 24 and FIG. 26 show that both the fusion proteins with triple amino acid mutations K26R-W31Y-Q23 and the fusion proteins with triple amino acid mutations K26R-W31Y-Y19 can activate the GLP-1 receptor.

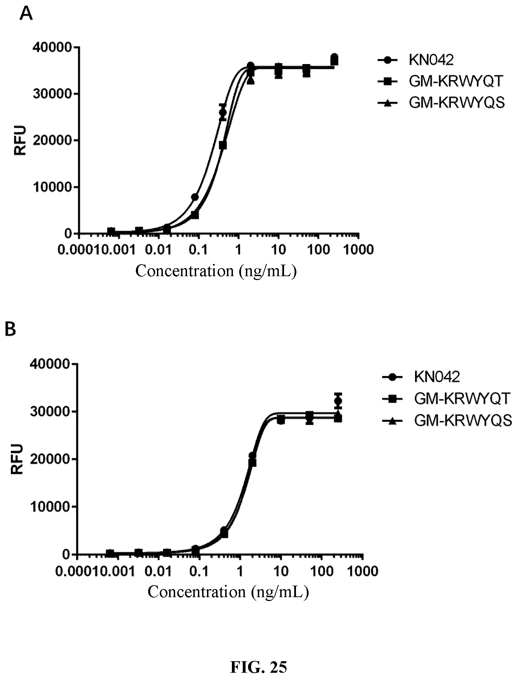

FIG. 25 shows that the fusion proteins with triple amino acid mutations K26R-W31Y-Q23 can activate the GLP-1 receptor.

### Example 4. Pharmacokinetic Assays

The *in vivo* pharmacokinetics of the GLP-1-Fc fusion proteins was investigated. The experiment was carried out in an SPF animal room at an ambient temperature of 23 ± 2 °C and 40-70% relative humidity, with alternate light and darkness (12 h). The experimental animals were given *ad libitum* access to food and water and, before the experiment, acclimated for at least 3 days. SPF SD rats weighing 180-220 g were randomly grouped. Into each group of rats, a corresponding test sample was subcutaneously injected, with dulaglutide as a control. Blood samples of about 100 µL were taken from the jugular vein before administration and 6 h, 24 h, 48 h, 72 h, 96 h and 120 h after administration. The blood samples were added to centrifuge tubes and left to stand at room temperature for 30-60 min. The blood was then centrifuged at 4 °C at 4000 rpm for 10 min within 2 h, and the serum was rapidly separated and stored at -80 °C. The samples were taken for ELISA analysis. The pharmacokinetic parameters were calculated using DAS (3.2.8) software.

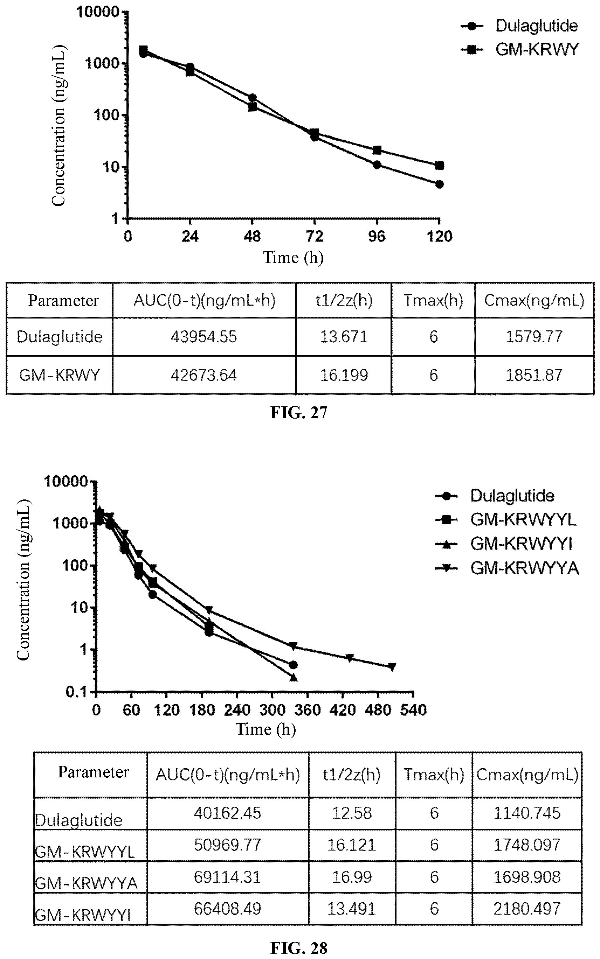

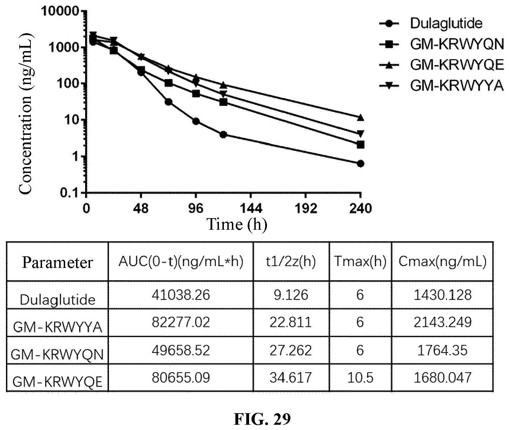

The results show that the fusion proteins of the present application have good pharmacokinetic properties compared to the control. FIG. 27 shows that the fusion protein GM-KRWY of the present application has a longer half-life (16.199 h) and higher drug exposure than dulaglutide and has a similar plasma concentration to dulaglutide. FIG. 28 shows that the fusion proteins GM-KRWYYL, GM-KRWYYA and GM-KRWYYI of the present application have longer half-lives (about 13 h to 16.5 h) than dulaglutide and have higher drug exposures and plasma concentrations than dulaglutide. FIG. 29 shows that the fusion proteins GM-KRWYQN, GM-KRWYQE and GM-KRWYYA of the present application have longer half-lives (about 22 h to 25 h) than dulaglutide and have higher drug exposures and plasma concentrations than dulaglutide.

### Example 5. Oral Glucose Tolerance Test (OGTT)

The *in vivo* hypoglycemic activity of the GLP-1-Fc fusion proteins was investigated. Mice were fasted overnight and were randomly grouped after the blood sugar and body weight were measured prior to the experiment. Into each group of mice, a corresponding test sample was intraperitoneally injected, with dulaglutide as a control. Each mouse was orally loaded with 1.5 g/kg glucose 0.5 h after administration. The mice were tested for tail-tip blood sugar level with a handheld Roche glucometer (Roche, Accu-chek) before glucose loading and 10, 20, 30, 60 and 120 min after loading. The mice's tail tips were cut off by about 1 mm, and the tails were squeezed from base to tip to collect blood from the veins. The blood was tested for blood sugar level.

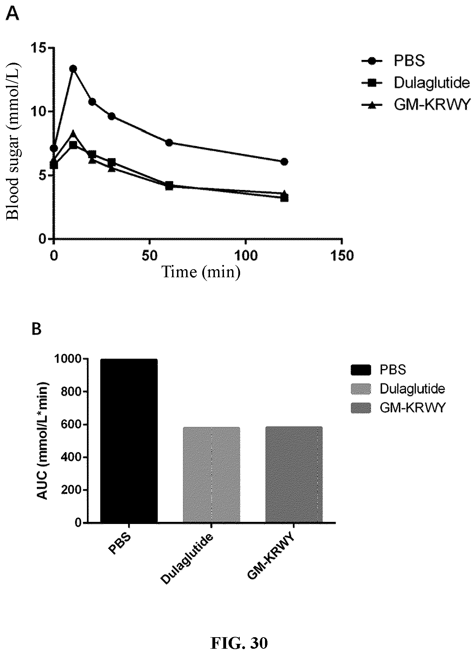

The results show that the fusion proteins GM-KRWY (FIGs. 30A and 30B), GM-KRWYYA and GM-KRWYQE (FIGs. 31A and 31B) of the present application have equivalent levels of *in vivo* hypoglycemic activity to the control.

## Claims

1. A human GLP-1 polypeptide variant, wherein the amino acid sequence of the human GLP-1 polypeptide variant comprises mutations at amino acid positions W31 and K26, and a mutation at an amino acid position selected from the group consisting of Q23 and Y19 compared to the amino acid sequence set forth in SEQ ID NO: 2.

2. The human GLP-1 polypeptide variant according to claim 1, having at least partial activity of human GLP-1.

3. The human GLP-1 polypeptide variant according to any one of claims 1-2, wherein the amino acid sequence of the human GLP-1 polypeptide variant comprises mutations at amino acid positions W31, K26 and Y19 compared to the amino acid sequence set forth in SEQ ID NO: 2.

4. The human GLP-1 polypeptide variant according to any one of claims 1-3, wherein the amino acid sequence of the human GLP-1 polypeptide variant comprises mutations at amino acid positions W31, K26 and Q23 compared to the amino acid sequence set forth in SEQ ID NO: 2.

5. The human GLP-1 polypeptide variant according to any one of claims 1-4, comprising an amino acid substitution at the position W31, wherein the amino acid substitution is W31Y, W31R, W31K or W31A.

6. The human GLP-1 polypeptide variant according to any one of claims 1-5, comprising an amino acid substitution at the position Y19, wherein the amino acid substitution is selected from any one of the following amino acid substitutions: Y19A, Y19L, Y19T, Y19F, Y19I, Y19V and Y19S.

7. The human GLP-1 polypeptide variant according to any one of claims 1-6, comprising an amino acid substitution at the position Q23, wherein the amino acid substitution is selected from any one of the following amino acid substitutions: Q23E, Q23T, Q23S and Q23N.

8. The human GLP-1 polypeptide variant according to any one of claims 1-7, comprising an amino acid substitution at the position K26, wherein the amino acid substitution is K26R.

9. The human GLP-1 polypeptide variant according to any one of claims 1-8, comprising an amino acid sequence set forth in SEQ ID NO: 150.

10. The human GLP-1 polypeptide variant according to any one of claims 1-9, comprising an amino acid sequence set forth in any one of SEQ ID NOs: 61-71.

11. A fusion protein or immunoconjugate, comprising the human GLP-1 polypeptide variant according to any one of claims 1-10.

12. The fusion protein or immunoconjugate according to claim 11, further comprising an immunoglobulin Fc region.

13. The fusion protein or immunoconjugate according to claim 12, wherein the immunoglobulin Fc region is an Fc region derived from IgG.

14. The fusion protein or immunoconjugate according to claim 11 or 12, wherein the immunoglobulin Fc region is an Fc region derived from IgG4.

15. The fusion protein or immunoconjugate according to any one of claims 11-14, wherein the immunoglobulin Fc region comprises an amino acid sequence set forth in any one of SEQ ID NOs: 139-143.

16. The fusion protein or immunoconjugate according to any one of claims 11-15, wherein the human GLP-1 polypeptide variant is fused in-frame with the immunoglobulin Fc region.

17. The fusion protein or immunoconjugate according to any one of claims 11-16, comprising a linker peptide.

18. The fusion protein or immunoconjugate according to claim 17, wherein the human GLP-1 polypeptide variant and the immunoglobulin Fc region are linked through the linker peptide.

19. The fusion protein or immunoconjugate according to claim 17 or 18, wherein the linker peptide comprises an amino acid sequence set forth in any one of SEQ ID NOs: 144-149.

20. The fusion protein or immunoconjugate according to any one of claims 11-19, comprising an amino acid sequence set forth in any one of 128-138.

21. An isolated nucleic acid molecule, encoding the human GLP-1 polypeptide variant according to any one of claims 1-10 or the fusion protein or immunoconjugate according to any one of claims 11-20.

22. A vector, comprising the isolated nucleic acid molecule according to claim 21.

23. A cell, comprising or expressing the human GLP-1 polypeptide variant according to any one of claims 1-10, the fusion protein or immunoconjugate according to any one of claims 11-20, the isolated nucleic acid molecule according to claim 21, or the vector according to claim 22.

24. A pharmaceutical composition, comprising the human GLP-1 polypeptide variant according to any one of claims 1-10, the fusion protein or immunoconjugate according to any one of claims 11-20, the isolated nucleic acid molecule according to claim 21, the vector according to claim 22, or the cell according to claim 23, and optionally a pharmaceutically acceptable adjuvant.

25. Use of the human GLP-1 polypeptide variant according to any one of claims 1-10 or the fusion protein or immunoconjugate according to any one of claims 11-20 for preparing a medicament for preventing and/or treating metabolic diseases or conditions.

26. The use according to claim 25, wherein the metabolic diseases or conditions include GLP-1-associated metabolic conditions.

27. The use according to any one of claims 25-26, wherein the metabolic diseases or conditions include diabetes.

28. A method for increasing or promoting insulin expression in a subject in need thereof, wherein the method comprises administering to the subject an effective amount of the human GLP-1 polypeptide variant according to any one of claims 1-10 or the fusion protein or immunoconjugate according to any one of claims 11-20.
